# EUROPÄISCHE PATENTSCHRIFT

(11) **EP 3 054 999 B1**
(45) Veröffentlichungstag und Bekanntmachung des Hinweises auf die Patenterteilung: **07.03.2018**
(21) Anmeldenummer: 14809758.7
(22) Anmeldetag: 09.10.2014
(51) Int. Cl.: A61L 27/18, A61L 27/50, A61F 2/30

(54) **GELENKSPACER**
JOINT SPACER
ESPACEUR ARTICULAIRE

(30) Priorität: 11.10.2013 DE 102013016899; 19.11.2013 DE 202013010444 U
(43) Veröffentlichungstag der Anmeldung: 17.08.2016
(60) Teilanmeldung: 18153478.5
(73) Patentinhaber: Revomotion GmbH, 50937 Köln (DE)
(72) Erfinder: JANSEN, Josef, 51429 Bergisch Gladbach (DE)
(74) Vertreter: Patentanwälte Vomberg & Schart
(86) Internationale Anmeldenummer: PCT/DE2014/100354
(87) Internationale Veröffentlichungsnummer: WO 2015/051785

(56) Entgegenhaltungen:
- US-A- 5 133 742
- US-A- 5 993 972
- US-A- 6 140 452
- US-A1- 2007 027 285
- US-A1- 2009 234 453
- US-A1- 2013 211 529
- US-A1- 2013 218 275

## Beschreibung

Die vorliegende Erfindung betrifft einen Gelenkspacer, insbesondere einen Knie-spacer und einen Hüftspacer.

Von Osteoarthrose, dem vorzeitigen Verschleiß knorpeliger Gelenkflächen, sind weltweit viele Millionen Menschen betroffen, der größte Teil davon an den Knien und an der Hüfte. Als Alternative zu den bisherigen mit vielfältigen Nachteilen verbundenen künstlichen Gelenken (Endoprothesen) bieten sich für das Knie elastische, scheibenförmige Abstandshalter, sogenannte "Kniespacer" an. Diese lassen sich mittels eines kleinen Hautschnitts nach der Entfernung des (Rest-) Meniskus, der Glättung der Gelenkflächen und einer sorgfältigen Größenauswahl in den Knieinnenraum einführen. Sie ersetzen im Gelenk den abgeriebenen Knorpel und den beschädigten Meniskus. Die Kniespacer stellen den natürlichen Gelenkspalt wieder her und können so auch Abwinklungen, wie beispielsweise X- oder O-Fehlstellungen (Valgus, Varus) des Beines, korrigieren.

Die natürlichen medialen (innen) und lateralen (außen) Menisken sind halbmondförmige Faserknorpelgewebe, die im Querschnitt keilförmig sind und über das Vorder- und Hinterhorn im Tibiaplateau verankert sind. Sie vergrößern die Kontaktfläche zwischen dem Tibiaplateau und den Gelenkrollen (Kondylen) des Femurs (Oberschenkelknochen).

Gelenkspacer bezeichnen allgemein Abstandshalter, die auch in anderen Gelenken, wie beispielsweise der Hüfte, der Schulter, dem Fuß, der Hand oder der Wirbel des menschlichen Körpers eingesetzt werden können. Gelenkspacer beinhalten hier auch sogenannte "Plugs" oder flache Stöpsel, welche nur einen kleinen Teil der Knorpelfläche eines Gelenks ersetzen können. Zudem sind Abstandshalter als Gleit- und Dämpfungselemente für Endoprothesen einsetzbar. Bei Kniespacern wird zwischen verschiedenen Ausführungsformen unterschieden. Der Meniskusspacer ersetzt den Meniskus und abgelaufenen Knorpel der Gelenkflächen und das Meniskusimplantat lediglich den Meniskus. Der Gelenkflächenersatz oder Gelenkflächenspacer gleicht den teilweise abgelaufenen Knorpel bzw. den beginnenden Verschleiß des Knorpels der Gelenkflächen ohne Ersatz des Meniskus aus.

Es ist bekannt, dass Kniespacer aus unterschiedlichen Materialien und Polymeren bestehen können. Dabei sind Polyurethane aufgrund ihrer hohen mechanischen Festigkeit und hohen Abriebfestigkeit sehr gut geeignet. Grundsätzlich kann bei Polyurethanen zwischen aromatischen und aliphatischen Polyurethanen unterschieden werden. Aromatische Polyurethane besitzen üblicherweise die größere mechanische Festigkeit und scheinen daher besser geeignet für die hohen Belastungen, die in einem Kniespacer während der Beugung und der Streckung auftreten. Aromatische Polyurethane haben gegenüber aliphatischen jedoch den Nachteil, dass sie "vergilben" können, also einen gewissen Alterungseffekt zeigen. Zudem stehen aromatische Polyurethane in der Diskussion, dass ihre potenziellen Abbauprodukte ein kanzerogenes Risiko besitzen können.

Kniespacer haben sich bis heute noch nicht durchsetzen können, weil die Implantate häufig brechen, aus dem Gelenkspalt des Knies herausrutschen oder Bewegungseinschränkungen auftreten. Insbesondere sind derzeit keine Kniespacer mit physiologischer Dämpfung bei gleichzeitig ausreichender mechanischer Festigkeit und Dauerfestigkeit bekannt. Insbesondere eine physiologische Dämpfung ist jedoch erstrebenswert, da sie zu einer Schmerzfreiheit des Patienten führt.

Unabhängig hiervon ist auf bekannte Gelenkspacer beziehungsweise Materialien zur Herstellung von Gelenkspacern hinzuweisen. Insbesondere wird in US 6,140,452 ein Biomaterial für die Reparatur von Knorpel beschrieben, wobei das ausgehärtete Material eine Shore-A-Härte von 70 bis 75 und eine Zugfestigkeit von 60 bis 75 Mpa aufweisen soll.

US 2013/0218275 A1 beschreibt ebenfalls ein Gelenkimplantat mit einer Härte von 60 A bis 100 A, das ergänzend ein Elastizitätsmodul zwischen 0,01 GPa bis 0,1 GPa ausweisen soll.

Weitere Materialien, die für die Herstellung von Gelenkimplantaten und/oder Prothesen geeignet sein sollen, werden in US 2007/0027285 und US 5,133,742 beschrieben.

Es ist die Aufgabe der vorliegenden Erfindung, einen haltbaren, ausreichend gedämpften und abriebfesten Gelenkspacer zu schaffen, der auch punktuell sehr hohe Lasten aufnehmen kann. Der Gelenkspacer sollte zudem eine möglichst hohe Kongruenz zwischen den artikulierenden Gelenkflächen erzielen und nicht aus dem Gelenkspalt herausrutschen können.

Diese Aufgabe wird durch den Gelenkspacer nach Anspruch 1 gelöst. Erfindungsgemäß besteht daher der Gelenkspacer zumindest teilweise aus einem Material, insbesondere einem Elastomer oder thermoplastischen Elastomer mit einer Shore-A Härte zwischen 20 und 77 und Zugspannungswerten bei Dehnung zwischen 20% und 60% , vorzugsweise bei 50% (im folgenden auch 50%-Zugspannung genannt), größer 3,8 N/mm², vorzugsweise größer 4,6 N/mm² und besonders bevorzugt größer 6 N/mm², und/oder einer Shore-A Härte bis 85 und Zugspannungswerten bei Dehnungen zwischen 20% und 60% , vorzugsweise bei 50%, größer 6 N/mm², vorzugsweise größer 7 N/mm² und besonders bevorzugt größer 8 N/mm². Angemerkt ist hier, dass unter dem Spannungswert bei einer bestimmten Dehnung nicht der Tangenten- oder Sekantenmodul verstanden wird. Das Material sollte außerdem bevorzugt kein Fließverhalten bis 50% Dehnung, vorzugsweise bis 70% Dehnung aufweisen.

Vorzugsweise weist das Material bei einer Shore-A Härte zwischen 20 und 77 Zugspannungswerte bei 100%-Dehnung (100%-Zugspannung) von mindestens 5 N/mm², vorzugsweise mindestens 6 N/mm² und besonders bevorzugt mindestens 7,5 N/mm², oder bei einer Shore-A Härte bis 85 Zugspannungswerte bei 100%-Dehnung von mindestens 7,5 N/mm², vorzugsweise mindestens 8,5 N/mm² und besonders bevorzugt mindestens 10,5 N/mm² auf.

Eine weitere Kenngröße für geeignete Materialien sind die Druckspannungen, auch wenn die Schädigung des Materials deutlich mehr von den auftretenden Zugspannungen hervorgerufen wird. Erfindungsgemäß besteht daher der Gelenkspacer ferner zumindest teilweise aus einem Material mit einer Shore-A Härte zwischen 20 und 77 und Druckspannungswerten bei Stauchungen zwischen 20% und 60%, vorzugsweise bei 50% (im folgenden auch 50%-Druckspannung genannt), größer 7,8 N/mm², vorzugsweise größer 9 N/mm² und besonders bevorzugt größer 10,5 N/mm², und/oder einer Shore-A Härte bis 85 und Druckspannungswerten bei Stauchungen zwischen 20% und 60%, vorzugsweise bei 50%, größer 10,5 N/mm², vorzugsweise größer 12 N/mm² und besonders bevorzugt größer 14 N/mm² bestehen.

Ein solcher Gelenkspacer zeigt vorteilhafterweise ein ausgeprägtes progressives Druck-Stauchungsverhalten. Mit zunehmender Stauchung nimmt nämlich die Elastizität überproportional ab und der Gelenkspacer wird mithin härter. Mit anderen Worten das Material ist hinsichtlich seiner Shore-Härte relativ weich und besitzt quer zur Stauchung gleichzeitig einen relativ hohen Elastizitätsmodul.

Die zuvor genannten Spannungswerte können auch bereits bei niedrigeren Dehnungs- bzw. Stauchungswerten als den genannten Werten erreicht werden, auch wenn der Gelenkspacer dann bei gleichen Belastungen nicht so stark zusammengestaucht wird.

Im Zusammenhang mit der Auswahl geeigneter Materialien bzw. Polymere für Gelenkspacer wird häufig die Shore-Härte herangezogen. Die in den Gelenken, besonders in Knie und Hüfte, auftretenden sehr hohen Belastungen können unter den bekannten für die Medizintechnik geeigneten Materialien nur durch Materialen mit Shore-A-Härten oberhalb von 85 aufgenommen werden. Für eine hohe Kongruenz und Dämpfung und letztlich damit Schmerzfreiheit für den Patienten wären Gelenkspacer aus solchen Materialien jedoch im Knie zu hart und daher weniger geeignet. Je weicher der Gelenkspacer, desto größer ist die Kontaktfläche zwischen Gelenkspacer und Gelenkfläche und desto kleiner die Spannung und damit auch der Abrieb im Gelenkspacer. Der Gelenkspacer sollte idealerweise in seiner Komplianz dem natürlichen Gelenkknorpel angenähert sein. Dies schließt den geschwächten arthrotischen Gelenkknorpel ein, so dass dieser nach Möglichkeit nicht weiter durch das Implantat geschädigt werden kann. Es wird ein Shore-A-Härte Bereich des Materials von 20 bis 77, vorzugsweise von 45 bis 72 angestrebt. Für sehr schwergewichtige Menschen, abhängig von der Größe der Tibiafläche bzw. des Gelenkspacers ab ca. 100-120 kg, kann darüber hinaus die Shore-A-Härte des Materials bis 85 liegen. Hierzu kommen als Materialien in erster Linie Elastomere oder thermoplastische Elastomere in Frage. Die bekannten flexiblen Polymere bzw. sonstigen Materialien in diesem Härtebereich können bei höheren Verformungen nicht genügend Spannung aufbauen, um den auftretenden Kräften in den Gelenken standzuhalten, d.h. die Tragfähigkeit des Lagermaterials reicht nicht aus. Es hat sich in Versuchsreihen jedoch gezeigt, dass Materialien, die ein ausgeprägt progressives Druck-Stauchungsverhalten aufzeigen, für Gelenkspacer geeignet sind. Materialien können nämlich bei gleicher Shore-Härte sehr unterschiedlich hohe Elastizitätsmodule aufweisen, und insbesondere bei einer vorgegebenen Dehnung oder Stauchung unterschiedlich hohe Spannungen aufbauen. Anders ausgedrückt wird unter einer vorgegebenen Last das Material bei gleicher Shore-A-Härte weniger oder mehr gestaucht.

Materialien, insbesondere Elastomere und thermoplastische Elastomere können bei Temperaturerhöhung, nach Aufnahme von Wasser und längerer, mehrmonatiger Lagerung in Wasser oder bei mehrfacher Belastung (belastungsinduzierte Entfestigung oder sogenannter Mullins-Effekt) erweichen (Geary C et al. Characterisation of Bionate polycarbonate polyurethanes for orthopaedic applications. J Mater Sci: Mater Med 19 (2008) 3355-3363). Zudem können sich durch Sterilisation die mechanischen Eigenschaften verändern. Weiterhin können sich Materialien bei sehr hohen Prüfgeschwindigkeiten verfestigen. Die in dieser Erfindung angegebenen Parameter beziehen sich daher auf Prüfbedingungen der Proben in einem konditionierten Zustand bei 37°C, nach Aufnahme von Wasser bzw. Gelenkflüssigkeit und mit den üblichen Prüfgeschwindigkeiten der einschlägigen Normen und in einem sterilisierten Zustand. Die Spannungswerte werden vorzugsweise nach dem 5. Zyklus ermittelt, um den Mullins Effekt auszugleichen, da sich die Größe der Hysterese insbesondere in den ersten Zyklen stark ändern kann. Zudem sollten die zuvor angegebenen Werte auch nach mindestens 5 monatiger Lagerung in Wasser erreicht bzw. überschritten werden. Die Materialien sollten also hydrolytisch möglichst stabil sein. Allgemeiner ausgedrückt, sollen die eingesetzten Materialien die Parameter auch nach längerer Implantation im Körper beibehalten.
Wie zuvor angedeutet, können weichere Materialien zumeist den in den Gelenken auftretenden Belastungen nicht standhalten. Neben den zuvor beschriebenen Zugspannungswerten bei 50%- oder auch 100%-Dehnung und den 50%-Druckspannungen ist die Weiterreißfestigkeit ein weiterer wichtiger Materialparameter. Je weicher das Material ist, desto höher sollte idealerweise die Weiterreißfestigkeit sein, mit anderen Worten soll sie umgekehrt proportional zur Shore-A-Härte stehen. Bezugswerte dieser Beziehung sind: bei einer Shore-A-Härte von 55 soll die Weiterreißfestigkeit größer 60N/mm (vorzugsweise größer 70 N/mm) sein, bei einer Shore-A-Härte von 75 größer 35 N/mm (vorzugsweise größer 40 N/mm). Über diese Bezugswerte soll ein linearer Verlauf über die Shore-A-Härte Bereiche bestehen. Die Weiterreißfestigkeiten beziehen sich auf Probenformen gemäß dem "Trouser Tear Test".

In den Unteransprüchen sowie nachfolgend werden bevorzugte Ausgestaltungen der Erfindung beschrieben.

Als Materialien für die Gelenkspacer eignen sich biokompatible und elastische, dauerfeste Polymere und hier, wie bereits ausgeführt, insbesondere die Klasse der Polyurethane. Allerdings können auch andere Werkstoffe verwendet werden, wie beispielsweise Silikone, PTFE, Polysulfone, Polyvinylalkohol, Poly[Styrol-Block-Isobutylen-Block-Styrol] (SIBS, poly[styrene-block-isobutylene-block-styrene]), hydrogenierte Styrene-Block-Copolymere (SBS, SIS, SEBS) oder andere aus dem Stand der Technik bekannte Polymere. Zudem sind neben diversen Kautschuken auch Seide bzw. künstliche Seide für die Gelenkspacer geeignet. Angemerkt sei ferner, dass auch Materialien mit hoher Wasseraufnahme in der Größenordnung von 100% und mehr als geeignete Werkstoffe für die Gelenkspacer in Frage kommen. Ferner sollen die Polymere möglichst frei sein von niedermolekularen Bestandteilen, wodurch die gewünschten 50%-Spannungswerte gesteigert werden und damit zu den bevorzugten Eigenschaften führen.

Vorzugsweise besteht das Elastomer oder thermoplastische Elastomer des Gelenkspacers aus der Klasse der Polyurethane und insbesondere aus einem Polyurethan, Polyharnstoff oder Polyurethanharnstoff.

Polyurethane sind charakterisiert durch das Zusammenspiel von Hart- (gebildet aus einem Isocyanat mit niedermolekularen Kettenverlängerern) und (höhermolekularen) Weichsegmenten. Abhängig von ihrer Zusammensetzung zeigen Polyurethane ein sehr unterschiedliches Werkstoffverhalten, insbesondere im Spannungs-/Dehnungsverlauf.

Isocyanate werden in der Literatur vielfältig beschrieben und können grundsätzlich alle eingesetzt werden. Bevorzugt werden jedoch kompakte, wenig verzweigte und hochschmelzende Isocyanate wie Cyclohexandiisocyanat (CHDI), Naphthalin-1,5-Diisocyanat (NDI), oder Para-Phenylendiisocyanat (PPDI). Ferner eignen sich lineare, symmetrische Isocyanate wie Hexamethylendiisocyanat (HDI). Derartige Ausgangskomponenten begünstigen weiter das progressive Druck-Stauchungsverhalten, woraus sich die genannten Vorteile ergeben. Zudem sind diese Isocyanate besonders gut geeignet für dynamische Anwendungen. Die Gewichtsanteile dieser Isocyanate betragen bis zu 50% vorzugsweise 3% - 30% der Polymere. Diese bevorzugten Isocyanate können ferner mit weiteren aromatischen oder aliphatischen Isocyanaten gemischt werden. Den zuvor genannten Diisocyanaten oder Mischungen aus Diisocyanaten können auch Polyisocyanate hinzugefügt werden.

Nach einer bevorzugten Ausgestaltung der vorliegenden Erfindung ist vorgesehen, dass das Polyurethangemisch zumindest teilweise trans-1,4-Cyclohexandiisocyanat (CHDI), cis-CHDI oder Mischungen hiervon besitzt. Trans-CHDI ist dabei besonders bevorzugt, weil das Polymer dadurch eine besonders hohe Kristallinität aufweist und damit die bevorzugten Eigenschaften erhält. CHDI führt zudem zu den favorisierten aliphatischen Polyurethanen. Ferner ist vorgesehen, dass das Polyurethangemisch zumindest teilweise aus weiteren aliphatischen Isocyanaten besteht, insbesondere aus Dicyclohexylmethandiisocyanat (H₁₂MDI) oder einem Isomerengemisch hiervon, oder auch einem Gemisch aus CHDI und H₁₂MDI.

Im Folgenden werden eine Reihe von Formulierungen bzw. Komponenten angegeben, die alle für sich vorteilhafterweise zu den bevorzugten Eigenschaften führen.

Zur Erzielung des gewünschten progressiven Druck-Stauchungsverhaltens von Polyurethanen mit hohen Spannungswerten bei 50%-Dehnung oder Stauchung und niedriger Shore-A-Härte sind die Weichsegmente von entscheidender Bedeutung. Vorzugsweise kommen für die Gelenkspacer hydrolysefeste und biostabile Weichsegmente auf Basis von Polyolefinen wie insbesondere Polyisobutylen (PIB) oder Polybutadien (PB) in Frage. Ferner gelten auch Weichsegmente auf Basis von Polycarbonat (PC) und Polydimethylsiloxan (PDMS) als relativ hydrolysefest. Typische aus der Literatur bekannte bzw. kommerzielle, auch medizinische Polyester- oder Polyetherurethane sind für Langzeitimplantate nicht genügend biostabil und kommen daher für die Gelenkspacer weniger bzw. nicht bevorzugt in Frage, auch wenn sie in relativ kleinen Anteilen beigemischt werden können. Auch Silikon-Ether-Polyurethan Copolymere scheinen eine ungenügende Biostabilität zu haben.

Nach einer bevorzugten Ausgestaltung der vorliegenden Erfindung ist das Weichsegment des Elastomers oder thermoplastischen Elastomers ein Polyisobutylen (PIB) bzw. ein bifunktionelles Polyisobutylen, bevorzugt ein hydroxylterminiertes (HO-PIB-OH) oder ein aminterminiertes (H₂N-PIB-NH₂) Polyisobutylen.

In einer anderen bevorzugten Ausgestaltung ist das Weichsegment Polybutadien (PB), bevorzugt OH-terminiertes und besonders bevorzugt OH-terminiertes hydrogeniertes Polybutadien. Es können jedoch auch aminterminierte Polybutadiene eingesetzt werden.

Die vorteilhaften Eigenschaften des Polymers werden besonders hoch, wenn das Weichsegment ausschließlich aus einem PIB oder PB oder auch aus einer Mischung dieser beiden Weichsegmente besteht.

Für das Weichsegment aminterminiertes PIB eignen sich als Kettenverlängerer Ethylendiamin (EDA) oder 1,4-Diaminobutan (BDA) und bevorzugt 1,6-Diaminohexan (HDA) oder 1,8-Diaminooctan (ODA). Das hydroxylterminierte PIB lässt sich besonders gut mit dem Kettenverlängerer 1,6-Hexandiol (HD) kombinieren. Bei Verwendung dieser Kettenverlängerer können die Weichsegmente jeweils sehr gut mit anderen Weichsegmenten gemischt werden, um das Werkstoffverhalten gezielt zu modifizieren und einzustellen. Vorzugsweise wird hierbei H₁₂MDI verwendet, es können jedoch auch die genannten anderen Isocyanate eingesetzt werden.

Das hydroxylterminierte PIB ergibt ferner mit dem Kettenverlängerer Butandiol ein geeignetes Polyurethan, wenn als Katalysator 1,3-Diacetoxy-1,1,3,3-Tetrabutyldistannoxan (DTDS) eingesetzt wird. Hierbei kann das Polymer auch in einem 1-Schritt-Polymerisationsverfahren synthetisiert werden, ohne das sogenannte Prepolymerverfahren, welches ansonsten in dem 2-Schritt-Verfahren bevorzugt verwendet wird. Als Isocyanat eignet sich neben den bevorzugten Isocyanaten besonders 4,4'-Diphenylmethandiisocyanat (MDI).

Das Weichsegment PB, insbesondere das hydroxylterminierte hydrogenierte Polybutadien lässt sich vorzugsweise mit den Kettenverlängerern N,N-Diisopropanol Anilin (DIPA) oder 2-Ethyl-1,3-Hexandiol (EHD), es gehen jedoch auch 2,2,4-Trimethyl-1,3-Pentandiol (TMPD) oder 2-Butyl-2-Ethyl-1,3-Propandiol (BEPG). Mit diesen Kettenverlängerern kann das Weichsegment wiederum mit anderen Weichsegmenten gemischt werden. Vorzugsweise wird hierbei MDI verwendet, es können jedoch auch die anderen genannten Isocyanate zur Anwendung kommen.

Polyurethane sind zudem besonders gut geeignet und führen zu den vorteilhaften Materialeigenschaften, wenn für die Polymerisation der Katalysator 1,3-Diacetoxy-1,1,3,3-Tetrabutyldistannoxan (DTDS) eingesetzt wird. Hierbei ist, wie zuvor ausgeführt, das 1-Schritt-Polymerisationsverfahren sehr gut geeignet.

Nicht zuletzt können die Polyurethantypen auch vernetzt sein. Hierfür eignet sich vorzugsweise Wasser für die Erzielung der gewünschten Eigenschaften. Es können jedoch weitere Vernetzungsreagenzien wie z.B. 3-wertiges Glycol oder sonstige mehrwertige aus der Polyurethanchemie an sich bekannte Vernetzungsreagenzien eingesetzt werden.

Bevorzugte Isocyanate bei der Wasservernetzung sind HDI, NDI, PPDI oder CHDI. Bevorzugte Weichsegmente sind hierbei neben den zuvor genannten bevorzugten Weichsegmenten PIB und PB auch Polycarbonatdiol (PCD) und zudem gegebenenfalls als Kettenverlängerer Butandiol (BD) oder ein oder mehrere andere Kettenverlängerer. Vorzugsweise wird jedoch auf einen weiteren Kettenverlängerer verzichtet, also nur Wasser zur Vernetzung eingesetzt.

Bei Polyurethanen auf Basis von Polycarbonatdiolen wird in der Medizintechnik üblicherweise Polyhexamethylencarbonatdiol (C₆-PCD) eingesetzt. Diese sind ausschließlich aus jeweils sechs Methylengruppen (CH₂) zusammengesetzt. Um jedoch weichere Polyurethane zu erzielen, zugleich aber die vorteilhaften Materialeigenschaften zu erzielen, die für die Gelenkspacer besonderes gut geeignet sind, werden Polycarbonatdiol-Copolymertypen (Polyalkylen-Carbonatdiole) statt eines homogenen C₆-PCD eingesetzt, die aus den Einheiten Hexan (C₆), Pentan (C₅), Butan (C₄) oder Propan (C₃) aufgebaut sind. Vorzugsweise bestehen die Copolymertypen aus den Kombinationen mit C₆/C₅ oder C₆/C₄ oder C₄/C₃ Einheiten, in der chemischen Summenformel ausgedrückt mit 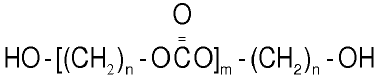 also mit n=6 oder 5; oder n=6 oder 4; oder n=4 oder 3. Zudem zeigen die Copolymertypen mit abnehmender Anzahl an Methylengruppen ein deutlich besseres Abriebverhalten.

Eine bevorzugte Formulierung sieht daher vor, dass das Polymer des Gelenkspacers zumindest teilweise Polycarbonatdiol-Copolymere enthält.

Besonders bevorzugte Komponenten des Polyurethansystem sind, wie bereits ausgeführt, die Isocyanate HDI, CHDI, PPDI oder NDI sowie als Weichsegmente Polyolefine, bevorzugt PIB oder PB und PCD, wobei bei PCD vorzugsweise die zuvor genannten Polycarbonatdiol-Copolymertypen eingesetzt werden. Hierbei wiederum können die Polyolefintypen PIB oder PB gemischt oder jeweils insbesondere mit dem PCD gemischt werden, um besonders abriebfeste Polyurethantypen zu erzielen. Der Anteil von PCD am Gesamtweichsegmentanteil beträgt bis zu 80%, vorzugsweise 5 bis 40%. Die Weichsegmente können ferner mit weiteren bekannten Weichsegmenten wie insbesondere Polydimethylsiloxan (PDMS) oder Polytetramethylenoxid (PTMO) gemischt werden. Auch eignet sich zur Erzielung der vorteilhaften Eigenschaften die Beimischung von Polydimethylsiloxan-Polycaprolacton-Blockcopolymeren. Die Anteile dieser Weichsegmente am Gesamtweichsegmentanteil betragen bis zu 50%, vorzugsweise 3 bis 35%.

Eine weitere vorteilhafte Formulierung sieht vor, dass das Polymer des Gelenkspacers als Weichsegmente ausschließlich hydroxyl- und/oder aminterminiertes Polyisobutylen, und/oder hydroxyl- und/oder aminterminiertes und bevorzugt hydroxylterminiertes hydrogeniertes Polybutadien, und/oder Polycarbonatdiol enthält.

Bei den zuvor genannten amin- oder hydroxylterminierten PIB kann es sich zudem um lineare oder verzweigte PIB's handeln. Bei den Polybutadienen können auch Copolymerisate wie Acrylnitril-Butadien-Styrol eingesetzt werden.

Ferner können auch die zuvor genannten Isocyanate mit anderen aromatischen oder aliphatischen bzw. cycloaliphatischen Isocyanaten gemischt werden, wie beispielsweise 3,3'-Dimethyl-4,4'-biphenylendiisocyanat (TODI).

Außerdem können in den zuvor aufgezeigten Formulierungen die genannten Kettenverlängerer auch in Kombination zweier oder mehrerer Kettenverlängerer und/oder Vernetzungsreagenzien (z. B. Glycol) in die Polymersynthese eingebracht werden.

Um die Zug-, die Weiterreiß- und insbesondere auch die Abriebfestigkeit des verwendeten Materials zu verbessern, ist nach einer vorteilhaften Ausgestaltung der Erfindung vorgesehen, dass das Material bzw. bevorzugte Polyurethangemisch Nanoadditive aufweist, die zumindest in einer Dimension wesentlich größere Abmessungen als in ihren beiden anderen Dimension aufweisen. Bevorzugt sind die Nanoadditive scheibenförmig oder flach ausgestaltet bzw. weisen in 2 Dimensionen wesentlich größere Abmessungen als in ihrer 3. Dimension auf. Vorzugsweise besitzen die Nanoadditive eine Breite von 10 nm bis 50 nm, vorzugsweise von 25 nm bis 30 nm, und eine Dicke von 0,5 nm bis 1,5 nm, vorzugsweise von 1 nm. Dadurch wird einerseits das progressive Druck-Stauchungsverhalten verbessert und andererseits die Kriechneigung verringert. Bei Nanoadditiven mit den angegebenen Abmessungen verhärtet das Material nicht, die Shore-Härte bleibt nahezu konstant, was aufgrund der verlangten Komplianz/Dämpfung des Gelenkspacers erwünscht ist. Durch die Zugabe der Nanoadditive erhöhen sich jedoch die 50%-Zug- oder Druckspannungswerte der Polymere. Die Nanoadditive werden bevorzugt in Volumenkonzentrationen von weniger als 10%, vorzugsweise weniger als 5%, weiterhin besonders bevorzugt weniger als 3%, hinzugefügt. Geeignete Materialien der Nanopartikel sind insbesondere geschichtetes Silikat, diverse Metalloxide, Kohlenstoff- oder Bornanopartikel, zudem auch Titan-, Platin-, Silber- oder Goldpartikel. Es können jedoch auch Kohlenstoff-Nanoröhrchen oder andere faserige Nanopartikel hinzugefügt werden. Ferner können prinzipiell auch langfaserige Verstärkungen im Material des Gelenkspacers eingearbeitet sein.

Mit den zuvor aufgezeigten Ausgestaltungen des Materials, d.h. je nach genauer Zusammensetzung der einzelnen Komponenten des Polyurethansystems und/oder der Zugabe von Nanopartikeln können 50%-Zugspannungen bis 20 N/mm² in den genannten Shore-A-Härtebereichen erzielt werden.

Zur Verbesserung der Gleiteigenschaften bzw. zur Reduzierung des Reibwertes ist vorgesehen, dass das Polyurethangemisch zudem Polyvinylpyrrolidon (PVP), Polyvinylalkohol (PVA) oder auch andere hydrophile Polymere bzw. dazu geeignete Substanzen aufweisen kann. Der Anteil dieser Substanzen liegt dabei unter 10%, vorzugsweise unter 3%.

In einer weiteren Ausführungsform der vorliegenden Erfindung besitzt zur Verbesserung des progressiven Druck-Stauchungsverhalten das verwendete Material, Polymer oder Polyurethangemisch eine Schaumstoff- bzw. Porenstruktur, wobei vorzugsweise das Material nach Konditionierung die zuvor genannten Spannungswerte bei 50%-Dehnung oder Stauchung in den relevanten Shore-A-Härte Bereichen aufweisen sollte. Die Porenstruktur kann grundsätzlich offenporig in Form im Wesentlichen einer Wabenstruktur oder geschlossen porig wie eine klassische Blasen- oder Schaumstoffstruktur sein. Versuche haben gezeigt dass Porengrößen in der Größenordnung von 200µm, wie sie häufig z.B. in Polyurethanschäumen auftreten, relativ schnell zu einer Schädigung des Gelenkspacers führen. Wesentlich besser geeignet sind Poren kleiner 5µm und nanoporöse Strukturen. Durch die Wasser- bzw. Gelenkflüssigkeitsaufnahme in die Poren werden auch schwächere Materialien, die die zuvor aufgezeigten Parameter nicht erfüllen, verstärkt und können damit höhere Lasten aufnehmen. Prinzipiell sind geschlossenporige Schaumstoffstrukturen für die Dauerfestigkeit vorteilhaft, jedoch haben gemischtzellige Strukturen den Vorteil, dass diese Flüssigkeiten aufnehmen können und der Flüssigkeitsaustausch zwischen den Zellen vereinfacht wird.

Vorteilhafterweise besitzt der Gelenkspacer daher zumindest teilweise eine Schaumstoffstruktur mit Porengrößen von 0,1 nm bis 2 µm, vorzugsweise von 1 nm bis 500 nm und besonders bevorzugt von 5 nm bis 200 nm. Größere Poren als 2 µm, insbesondere 5 µm führen zumeist zu einem frühzeitigen Verschleiß bzw. zur Schädigung des Gelenkspacermaterials. Zudem halten insbesondere offene Porenstrukturen mit Porengrößen kleiner 100 nm bis 200 nm Bestandteile, wie z.B. Proteine der Synovialflüssigkeit zurück, um eine Verhärtung oder auch Verkalkung der Strukturen zu vermeiden. Die poröse Struktur hat ferner den Vorteil, dass sich aufgrund der Flüssigkeitsaufnahme in den Poren auf der Oberfläche bei Belastung ein Tragfilm (Flüssigkeitsreibung) aufbauen kann. Anders ausgedrückt kann bei porösen Oberflächen eine Depotschmierung entstehen. Der Gelenkspacer soll daher an der Oberfläche vorzugsweise offenporig sein. Der Gelenkspacer kann jedoch auch kaschiert sein, d.h. an der Oberfläche ohne Poren vorliegen. Zur Verbesserung der Gleiteigenschaften bzw. um eine Flüssigkeitsreibung zu erzielen, kann auch nur die Oberfläche des Gelenkspacers ganz oder teilweise porös ausgebildet oder aufgeraut sein.

Die zuvor genannten Stoffe zur Verbesserung der Gleiteigenschaften bzw. zur Reduzierung des Reibwertes, wie Polyvinylpyrrolidon (PVP), Polyvinylalkohol (PVA) oder weitere geeignete Substanzen, können auch genutzt werden, um die Poren der Schaumstoffstruktur zu füllen oder zumindest zu benetzen und damit eine Depotschmierung zu erzielen.

Wie zuvor beschrieben, verbessern Nanopartikel sowie poröse Strukturen das progressive Druck-Stauchungsverhalten. Darüber hinaus verstärken sich die erzielten Effekte bei Kombination aus Poren und Nanopartikeln und es lassen sich besonders hohe 50%-Zug- oder Druckspannungswerte bei niedrigen Shore-A-Härten für Gelenkspacer realisieren.

Eine andere Ausführung zur Erzielung der gewünschten vorteilhaften Materialeigenschaften sieht eine poröse Vlies Struktur oder insbesondere Nano-Vlies Struktur vor, die vorzugsweise aus Fasern mit einem Durchmesser von 0,1 µm bis 0,4 µm besteht. Das Material besitzt eine feinfibrilläre Struktur (Wirrvlies) aus Fasern, die vorzugsweise gesponnen werden und an ihren Kreuzungspunkten gefügt bzw. miteinander verschmolzen sind. Weiterhin sollten die Poren einer solchen Vliesstruktur vorzugsweise kleiner 5µm und besonders bevorzugt kleiner 1 µm sein.

Der Vollständigkeit halber sei hinzugefügt, dass das progressive Druck-Stauchungsverhalten des Gelenkspacers mit schaum- oder vliesartiger Struktur und den oben angeführten Shore-A-Härten und 50%-Zug- oder Druckspannungen vorzugsweise erreicht wird, wenn die verwendeten Polymere in homogener oder kompakter Form deutlich härter sind. Anders ausgedrückt können auch Materialien oder Polymere mit Shore-A Härten größer als 77 oder 85 für einen Gelenkspacer besonders gut geeignet sein, wenn sie durch eine poröse Struktur weicher werden und damit die gewünschten Shore-A-Härten erreichen.

Insbesondere bei Kniespacern wird aufgrund der Konvexität der Kondylen des Femurs der zentrale Bereich des Gelenkspacers stark belastet. In Literatur und Patentschriften werden jedoch Kniespacer beschrieben, deren zentraler Bereich weicher und deren Randbereich härter ausgeführt sind. Allerdings neigen Kunststoffe zum Kriechen, wodurch der zentrale Bereich des Gelenkspacer bei dauernder Belastung "ausgebügelt" wird, also zentral wellig wird bzw. Falten werfen kann, die insgesamt zu Verwerfungen und zu Luxationen des Gelenkspacers führen können. Zudem können so die Randbereiche mit der Zeit immer weniger Last aufnehmen. Um einen Gelenkspacer mit den bevorzugten Dämpfungseigenschaften (Komplianz) zu erhalten, ist in einer weiteren Ausführungsform vorzugsweise zentral härteres und in den Randbereichen des Gelenkspacers weicheres Material angeordnet. Der Meniskusspacer ist so ausgelegt, dass zuerst die weicheren Randbereiche durch den Femur und anschließend der härtere zentrale Bereich belastet werden, wodurch das progressive Druck-Stauchungsverhalten erreicht wird. Die femurale Seite des Meniskusspacers ist dabei konkaver ausgebildet als die Konvexität des Femurs, d.h. bei Kontakt werden zuerst die Randbereiche des Meniskusspacers durch den Femur berührt.

Vorzugsweise werden hierbei die zuvor aufgezeigten härteren bzw. weicheren Materialien mit den entsprechenden 50%-Zug- oder Druckspannungswerten eingesetzt. Diese Ausführung kann aber auch aus bekannten Polyurethanen aufgebaut sein. Als hartes Material vorzugsweise mit dem Shore-A-Härtebereich zwischen 78 und 85 kann beispielsweise ein Material mit den niedrigeren 50%-Zug- oder Druckspannungswerten des Shore-A-Härtebereichs bis 77 genutzt werden. Zudem kann als weiches Material für den Randbereich ein Material mit niedrigeren 50%-Zug- oder Druckspannungswerten als den angegebenen des Shore-A-Härtebereichs 20-77 eingesetzt werden. Vorzugsweise verlaufen die weichen Bereiche keilförmig von Rand des Meniskusspacers in die Mitte und sind beidseitig vom harten Material eingebettet, wobei zentral nur das harte Material vorliegt. Die Verformung der weichen Randbereiche und damit der Erhalt der Rückstellung dieser Bereiche werden begrenzt durch den zentralen harten Bereich, welcher die volle Last aufnimmt.

Weiterhin kann der Gelenkspacer einen schichtartigen Aufbau mit mindestens drei Schichten aufweisen, nämlich zwei Deckschichten und einer dazwischen angeordneten Kernschicht. Dabei bestehen die Kernschichten und die Deckschicht aus unterschiedlich harten Materialien, wobei vorzugsweise die Kernschicht aus einer vergleichsweise härteren und die Deckschichten aus einer vergleichsweise weicheren Schicht bestehen. Die relativ weichen Randbereiche sind vorzugsweise in ihren Dicken entsprechend so bemessen, dass zunächst diese Bereiche von einer Kondyle belastet werden, womit der Gelenkspacer zunächst eine ausnehmend hohe Komplianz aufweist. Mit zunehmender Stauchung der flexiblen Randbereiche verhält sich der Gelenkspacer aufgrund der vergleichsweise härteren Kernschicht steifer (progressives Druck-Stauchungsverhalten).

Vorzugsweise ist die Differenz der Shore-Härte zwischen Deck- und Kernschicht größer als 5 und vorzugsweise zwischen 10 und 25. Die härtere Kernschicht besteht vorzugsweise aus einem Polymer mit einer Shore-A-Härte von 78 bis 90, vorzugsweise von 80 bis 84. Demgegenüber besitzen die Deckschichten eine Shore-A-Härte zwischen 20 und 77 und bevorzugt zwischen 40 und 70. Die harten bzw. weichen Schichten weisen 50%-Zug- oder Druckspannungswerte auf wie in der Ausführungsform zuvor mit weichem Rand beschrieben.

Auf eine der beiden weichen Deckschichten, insbesondere auf die distale, die - im Falle eines Kniespacers - zur Tibia (Schienbein) weist, kann auch verzichtet werden, bevorzugt dann, wenn der Kniespacer auf der Tibia fixiert wird. Der Vorteil der zusätzlichen weichen Schicht besteht jedoch darin, dass sich diese besser an die individuelle Gelenk-Topografie des Patienten anpassen kann. Angemerkt sei ferner, dass das progressive Druck-/Stauchungsverhalten grundsätzlich auch erzielt werden kann, wenn die Kernschicht weich und die Deckschichten hart sind.

Vor allem bei sportlichen bzw. mobilen Patienten ist zu beobachten, dass Gelenkspacer verrutschen können (Luxation) und nicht mehr die optimalen Lagen im Gelenk einnehmen. Insbesondere ist ein solches Risiko bei Gelenkspacern hoch, die als Kniespacer ausgebildet sind, da im Kniegelenk relativ wenig Platz für eine ausreichende Fixierung ist und kein kraftschlüssiger Formschluss erzielt werden kann, anders als bei einem Gelenkspacer für den Hüftkopf. Um das Risiko einer Luxation zu verringern, ist für die Gelenkspacer und insbesondere für die Kniespacer vorgesehen, dass sie entlang ihres Randes zumindest teilweise mit einer porösen Vlies-schicht oder Band aus feinfibrillärer Struktur bedeckt sind. Die feinfibrilläre Struktur (Wirrvlies) hat den Vorteil, dass hierin umliegende Zell- bzw. Gewebestrukturen einwachsen können. Der Meniskusspacer kann so insbesondere mit der Kapsel des Knies verwachsen und zur Positionierung und Luxationsreduzierung im Knie beitragen. Die feinfibrilläre Struktur besteht bevorzugt aus Nanofasern mit Durchmessern ≤ 0,4 µm oder aus dickeren Fasern mit mittleren Durchmessern von ca. 2 µm. Die feinfibrilläre Randschicht kann die Randfläche des Meniskusspacers in der gesamten Höhe oder auch nur teilweise bedecken. Dabei kann sie vollflächig mit der Randfläche des Meniskusspacers verbunden sein. Vorzugsweise ist allerdings die feinfibrilläre Randschicht jeweils nur am distalen und proximalen Rand mit dem Kniespacer verbunden. Vorteilhaft ist hierbei, dass es zu kleinen Relativbewegungen und damit zu einem Bewegungsausgleich zwischen Meniskusspacer und der angewachsenen feinfibrillären Randschicht kommt, womit die Positionierung des Meniskusspacers weiter unterstützt wird.

Nach einer weiteren bevorzugten Ausgestaltung der vorliegenden Erfindung umfasst zumindest teilweise ein Schlauch aus feinfibrillärer Struktur den Gelenkspacer, in dem ein zusätzliches Fixierungsband angeordnet ist. Der Schlauch ist vorzugsweise vollflächig an der Randfläche des Gelenkspacers befestigt. Das Fixierungsband, oder auch nur der Schlauch oder das Band aus feinfibrillärer Struktur ohne das zusätzliche Fixierungsband dienen dazu, den Gelenkspacer mit einem Gelenkpart, im Falle des Kniespacers mit den Resten der Meniskushörner oder anderen Stellen im Tibiaplateau, zu verbinden. Die feinfibrilläre Struktur besteht bevorzugt aus dem gleichen Material wie der eigentliche Gelenkspacer, so dass beide vorzugsweise mittels eines Diffusionsklebstoffs miteinander verbunden sind.

Die feinfibrilläre Umrandung kann auch zum Annähen des Meniskusspacers an den Resten des Meniskus bzw. der Gelenkkapsel genutzt werden. Alternativ oder ergänzend hierzu kann der Meniskusspacer auch einzelne oder eine Vielzahl von kleinen Bohrungen - vorzugsweise entlang des äußeren Randes - haben, die durch die gesamte Dicke des Meniskusspacers oder jeweils von der tibialen und/oder femuralen Fläche zur Randfläche führen. Angemerkt sei, dass die feinfibrilläre Umrandung auch andere faserige Strukturen aufweisen kann, beispielsweise gewebte, gestrickte oder gewirkte Strukturen. Zudem können in dem Gelenkspacer eingelassene Nahtfäden durch die faserige Umrandung nach außen geführt werden. Diese nach außen geführten Fäden sind bevorzugt anterior, am äußeren Rand oder auch posterior angebracht und können zur zusätzlichen Fixierung des Gelenkspacers mit den umliegenden Gewebestrukturen vernäht werden.

Nach einer alternativen Ausgestaltung ist zur Verhinderung einer Luxation vorgesehen, dass der zuvor erwähnte Schlauch lediglich um den äußeren kreisbogenförmigen Rand befestigt oder stoffschlüssig verbunden ist und diesen Schlauch dabei über die Enden des Kreisbogens zum Innern des Knies zu verlängern. Der Schlauch bildet in der Aufsicht eine C-Form. Im Schlauch verläuft ein flexibles und hochfestes Fixierungsband, welches beispielsweise aus Fasern aus ultrahochmolekulargewichtigem Polyethylen (UHMWPE) oder einem anderen Polymer - beispielsweise aus einem Polyurethan - besteht. Das Fixierungsband kann auch an den entsprechenden Stellen aus dem Schlauch bzw. der Vliesumrandung durch Öffnungen hinausgeführt werden, wenn der Schlauch um den vollen Umfang der Randfläche des Meniskusspacers umfasst. In einer Variante dieser Ausgestaltung ist der Schlauch mit innenliegendem Fixierungsband oder ggf. nur das Fixierungsband nicht mit dem Meniskusspacer stoffschlüssig verbunden, wodurch der Meniskusspacer sehr einfach auszutauschen ist. Der Meniskusspacer wird so nach Befestigung des Fixierungsbandes in die C-förmige Umrandung des Schlauches oder Fixierungsbandes eingelegt und so durch dieses nach außen kraft- bzw. formschlüssig gehalten.

Vorzugsweise wird das Fixierungsband mit den Resten der Meniskushörner verbunden. Dies kann mittels Annähen, über selbstschließende Clips oder verschließbare und ggf. wieder lösbare Klemmen bzw. Kupplungen erfolgen. Die Klemmen bzw. Kupplungen bestehen beispielsweise aus einer Form-Gedächtnislegierung. Die Klemme oder Kupplung, die mit der einen Hälfte mit dem Band fest verbunden ist, wird an der anderen Hälfte im Knie mit einer Zange zusammengepresst. Die Klemme könnte hierbei backenförmig mit Krallen ausgebildet sein, so dass sie sich gut in die Meniskushörner "verbeißen" kann. Sie kann bei einem wiederholten Eingriff über eine Temperaturerhöhung oberhalb der Körpertemperatur wieder geöffnet werden. Nach einer bevorzugten Ausführungsform könnte die Klemme bzw. Kupplung aus einer Form-Gedächtnislegierung auch durch einen Temperaturimpuls (ohne aktive Pressung mittels einer Zange) geschlossen werden. Eine weitere Variante besteht darin, eine Hälfte der Kupplung fest mit dem Meniskushorn und die andere Hälfte mit dem Fixierungsband zu verbinden. Der feinfibrilläre Schlauch kann nach Fixierung des Bandes mit den Meniskushörnern über die Verbindungsstelle geschoben werden. Hierzu kann der Schlauch auch an den Enden geschlitzt ausgebildet sein, um ihn besser über die Verbindungsstelle, d.h. über die Klemme oder die Kupplung führen zu können. Die geschlitzten Schlauchenden können nach dem Überschieben ggf. über einen Klettverschluss wieder (distal) fixiert werden.

Für den Fall, dass die Fixierung über die Meniskushörnerreste nicht möglich ist, kann das Band in Bohrungen im Tibiaplateau fixiert werden, wobei die Bohrungen bevorzugt in die Einmuldungen (Area intercondylaris anterior bzw. posterior) des Tibiaplateaus, genauer in die Ansatzpunkte des Vorder- bzw. Hinterhorns, eingebracht werden. Hierzu könnte das Fixierungsband an den Enden, ggf. über lösbare Klemmen, mit stiftförmigen Bauteilen verbunden sein. Auf gleiche Weise kann auch das Meniskusimplantat fixiert werden.

Nach einer weiteren bevorzugten Ausführungsform ist vorgesehen, dass das Fixierungsband in einer umlaufenden Nut in der Kernschicht des Gelenkspacers verläuft und aus der feinfibrillären Randschicht hinausführt. Das Fixierungsband ist in der Nut vorzugsweise frei beweglich oder in der Kernschicht fixiert. Hierbei ist die Kernschicht zum äußeren Rand verdickt ausgebildet, was die Aufhängung durch das Fixierungsband verstärkt. Alternativ kann das Fixierungsband auch nur um die Randfläche des Meniskusspacers angeklebt oder anders befestigt sein. Die feinfibrilläre Vliesschicht ist dann jeweils am distalen und proximalen Rand mit der Randfläche verbunden.

Ferner ist gemäß einer weiteren Ausführung vorgesehen, dass das Band in einer U- oder L-förmigen Profilschiene verläuft, so dass der Gelenkspacer lösbar vom Fixierungsband gelagert ist. Vorzugsweise ist die U-förmige Profilschiene mit der Kernschicht des Gelenkspacers verbunden. Da das Fixierungsband nach den beschriebenen Fixierungsmethoden dauerhaft im Knie verbleiben kann, muss bei einer Reoperation vorteilhafterweise nur der Gelenkspacer ausgetauscht werden, wohingegen das Fixierungsband im Körper des Patienten verbleiben kann.

Das U- oder auch L-förmige Profil ist vorzugsweise mit der festen Kernschicht des Gelenkspacers verbunden und an der Außenseite mit einer feinfibrillären Struktur bedeckt. Das Profil besteht nach einer vorteilhaften Ausgestaltung der Erfindung aus einem besonders harten Material, einem hochfesten Kunststoff oder Polyurethan oder auch einem Metall. Das außerhalb des eigentlichen Gelenkspacers liegende Fixierungsband sowie die Verbindungsstelle mit dem Meniskushorn können mit der feinfibrillären Schlauchstruktur umschlossen sein. Die Nut könnte auch in der Sandwichstruktur des Meniskusspacers integriert sein.

Die um den Knie-Spacer umlaufende weiche Umrandung, die feinfibrilläre Struktur, der Schlauch oder das Fixierungsband eignet sich zudem bevorzugt für die Befestigung von Markern, wodurch der Knie-Spacer bzw. dessen Position und Bewegung mittels bildgebender Verfahren sichtbar gemacht werden kann. Die Marker können kleine Pins, feine Fäden oder Drähte oder auch eine ferromagnetische Schicht sein, die vorzugsweise mit der weichen bzw. äußerst flexiblen Struktur der Umrandung verbunden sind. Auch kann die Umrandung oder das Fixierungsband selbst aus röntgendichten oder einem anderem für das jeweilige bildgebende Verfahren geeignetem Material bestehen. Die Befestigung der Marker an der Umrandung ist besonders vorteilhaft, da dadurch die Verformung des Gelenkspacers am wenigsten behindert werden kann. Wären die Marker im Gelenkspacer direkt befestigt, bestünde die Gefahr, dass sich diese mit der Zeit durch die dynamischen Belastungen lösen und in die Gelenkspalte eindringen.

Nach einer weiteren vorteilhaften Ausgestaltung besitzt der Kniespacer zwei Auskragungen, die am inneren Rand angeordnet sind und form- oder kraftschlüssig in die Einmuldungen oder Bohrungen greifen und so den Kniespacer auf dem Tibiaplateau fixieren. Diese (in einer Frontalansicht) L-förmigen Auskragungen bestehen vorzugsweise aus demselben Material wie die Kernschicht des Gelenkspacers und sind mit dieser stoffschlüssig verbunden. Die Auskragungen oder Teilbereiche der Auskragungen können alternativ auch aus einer feinfibrillären oder einer anderen geeigneten Struktur bestehen, die ein Annähen der Auskragungen mit den Meniskushörnern ermöglichen. Die Auskragungen sind insbesondere so ausgelegt, dass sie nur nach innen führen und nicht über das anteriore oder posteriore Ende des Tibiaplateaus hinausgehen. Sie behindern lediglich eine seitlich nach außen gerichtete Verschiebung des Gelenkspacers, jedoch keine Bewegung in anteriorposteriorer Richtung. Daher können sie auch elastisch ausgelegt sein, so dass die anterior-posteriore Bewegung des Kniespacers unterstützt wird. Nach einer bevorzugten Ausgestaltung sind die Auskragungen an ihren Enden stiftförmig ausgestaltet und in vorgefertigte Bohrungen einführbar, die an den Einmuldungen ausgebildet sind, was eine Luxation des Gelenkspacers verhindert. Die Übergänge der Auskragungen zur Grundfläche des Kniespacers können auch gerundet und in dessen Kontur integriert sein. Die Auskragungen können auch mit den Resten der Meniskushörner oder den Kreuzbänder verbindbar sein, wobei hierbei bevorzugt der distal gerichtete Teil der L-Auskragung fehlt oder deutlich vermindert ausgebildet ist.

Ferner sei angemerkt, dass natürlich die aus der Patentliteratur bzw. dem Stand der Technik bisher bekannten und beschriebenen Verfahren und Elemente zum Fixieren mit dem erfindungsgemäßen Kniespacer kombiniert werden können.

Neben den zuvor beschriebenen Fixierungsmethoden hat auch die Formgebung des Meniskusspacers wesentlichen Einfluss auf das Luxationsrisiko. Nach dem Stand der Technik sind Meniskusspacer aus flexiblen Materialien am äußeren Rand dicker als im zentralen Bereich, da der in der Aufsicht C-förmige und im Querschnitt keilförmige Meniskus bzw. Meniskusersatz in dem Spacer integriert ist. In Versuchen hat sich jedoch herausgestellt, dass diese Form des Meniskusspacers leicht zu Luxationen führt. Daneben gibt es noch im wesentlich gleichmäßig dicke Kniespacer, wie auch Meniskusspacer mit Verankerungsrippen auf der Tibiaseite des Kniespacers, die in entsprechende Ausbrüche der Tibia greifen bzw. mit diesem fixiert werden. Um das Luxationsrisiko zu vermeiden bzw. zu verringern, ist der Meniskusspacer in einer weiteren alternativen Ausgestaltung anterior und posterior dicker ausgestaltet als im zentralen Bereich des Meniskusspacers und zumindest in einem Bereich am äußeren Rand gleich dick oder bevorzugt dünner als der zentrale Bereich des Meniskusspacers, wobei sich der Bereich vom Mittelpunkt der kreisförmigen Randkontur aus gesehen um einen Winkel von 90°, vorzugsweise 45°, erstreckt. Besonders bevorzugt liegt die Stelle direkt posterior der Mitte des Randes. Darüber hinaus kann der zentrale äußere Rand in dem zuvor beschriebenen Bereich die geringste Höhe des gesamten Randes des Meniskusspacers aufweisen. Selbstverständlich kann diese Gestaltmodifikation mit den zuvor beschriebenen Ausführungen zur Fixierung der Kniespacer kombiniert werden, um das Luxationsrisiko weiter zu reduzieren.

Zur Implantation der Kniespacer wird folgendes bevorzugtes Verfahren angewandt. Nach Öffnung des Knieinnenraumes wird das Fixierungsband mittels der zuvor beschriebenen Möglichkeiten mit dem Tibiaplateau verbunden. Anschließend wird der Kniespacer in die durch das Fixierungsband gebildete C-förmige Mulde eingeführt. Hierbei kann der Kniespacer in seine Aussparungen (Nuten, Profile) formschlüssig eingefädelt oder innerhalb des Fixierungsbandes nur eingelegt werden. Nach einer bestimmten Zeitspanne kann der Kniespacer durch einen neuen ausgetauscht werden. Dazu müssen gegebenenfalls die zuvor beschriebenen Klemmen geöffnet werden.

Nach einer Weiterbildung der Erfindung ist der Gelenkspacer als Hüftspacer ausgestaltet und in Form eines schalenförmigen elastischen Überzugs über den Hüftkopf stülpbar. Vorteilhafterweise ist ein solcher Hüftspacer ohne jegliche Knochenresezierung minimal-invasiv einsetzbar. Vorteilhafterweise besitzt der Hüftspacer - bevorzugt in Nähe des proximalen Pols - eine Öffnung für die Durchführung des Hüftkopfbandes.

Der Hüftspacer ist nach einer bevorzugten Ausgestaltung der Erfindung von der Öffnung bis zum Rand des schalenförmigen Überzugs aufgetrennt, wobei entlang der Trennungslinie ein Überlappungsbereich vorgesehen ist. Mit anderen Worten der Hüftspacer ist nach unten (caudal) ähnlich eines "Hosenbundschlitzes" aufgetrennt, um den Hüftspacer um den Hüftkopf stülpen zu können, wenn das Hüftkopfband nicht abgetrennt wurde. Vorzugsweise weist der äußere Teil des Überlappungsbereiches nach innen eine Vielzahl von Noppen auf, die korrespondierende Ausnehmungen des unteren Teils des Überlappungsbereichs einführbar sind. Dabei sind die Noppen vorzugsweise entlang eines Medianbogens angeordnet. Alternativ hierzu sind auch andere Verbindungsmittel in Form von "Nippeln", "Knöpfen" bzw. Druckknöpfen, Schnüren oder andere geeignete Vorrichtungen zum Schließen und Fixieren vorgesehen, wie beispielsweise ein Klettverschluss oder formschlüssige Verbindungen mit Scharniergelenken oder Schnapphaken bzw. sonstige Schnappverbindungen. Insbesondere eignet sich hierbei eine sogenannte schräge Hakenblattung, wie man sie aus Holzverbindungen kennt. Auch eine stoffschlüssige Verbindung durch Kleben oder Schweißen kommt in Betracht. Ferner kann die Trennung des Hüftspacers mittels Tackern oder Heftklammern geschlossen werden. Weiterhin ist ein Reißverschluss oder auch Verzahnung aus Zinken eine geeignete Verbindung. Vorzugsweise ist die reißverschlussartige Verbindung in Form einer Verzahnung in Puzzleform ausgeführt.

Da der Hüftspacer vorzugsweise elastisch ausgeführt ist, kann er bezogen auf den Hüftkopf ein leichtes Untermaß aufweisen, um den Hüftspacer form- und kraftschlüssig mit dem Hüftkopf zu verbinden.

In einer alternativen Ausgestaltung kann der Hüftspacer proximal geschlossen und ohne Trennung ausgebildet sein, wenn das Hüftkopfband abgetrennt wurde. Allerdings kann für diesen Anwendungsfall der Hüftspacer vom Äquator bis zum freien Rand aufgetrennt sein, um ein Überstülpen über den Hüftkopf zu erleichtern. Die Trennung kann in Form eines einfachen Schnittes oder mit einem Überlappungsbereich in Form z.B. des Hosenbundschlitzes ausgeführt sein.

Die freien Ränder der beiden Pole des Hüftspacers können ganz oder teilweise aus einer porösen Vliesschicht aus feinfibrillärer Struktur bestehen. Alternativ kann auch ein Band oder wie beim Kniespacer ausgeführt ein Schlauch aus feinfibrillärer Struktur mit dem freien Rand verbunden sein, die gegebenenfalls über die Enden des freien Randes hinausgehen. Das feinfibrilläre Band selbst oder ein im Schlauch befindliches Fixierungsband kann zur Befestigung des Hüftspacers um den Hüftkopfhals durch z. B. Verknotung der Enden der Bänder dienen.

Die für den Hüftspacer geeigneten Materialien und Materialstrukturen entsprechen denen der Gelenkspacer bzw. des Kniespacers und damit den vorherigen Beschreibungen. Insbesondere kann der Hüftspacer auch teilweise aus degradierbaren Material oder einer Matrix bestehen, in die umliegende Zell- und Gewebestrukturen einwachsen und das Material bzw. die Matrix ersetzen oder auch ergänzen. Die Form und das Konzept des Hüftspacers können zudem aus gewebegezüchtetem Material bestehen, also mittels Tissue-Engineering hergestellten Knorpelgewebes gefertigt sein.

Der Hüftspacer ist - ähnlich wie der Kniespacer - aus homogenem oder porösem Material hergestellt und/oder mehrschichtig aufgebaut. Vorzugsweise ist eine Sandwichstruktur aus einer härteren inneren Kernschicht und weicheren äußeren Schichten vorgesehen. Es kann jedoch auch nur eine innere Kernschicht und eine äußere weiche Deckschicht verwendet werden. Die zum Hüftkopf gerichtete innere Seite kann sich jedoch besonders gut der Topografie des individuellen Hüftkopfpatienten anpassen, wenn sie besonders weich ist. Die Innenseite des Hüftspacers könnte zudem mittels eines geeigneten Klebers an den Hüftkopf befestigt werden. Beispielsweise kann die Unterseite mit Knochenzement unterspritzt werden um Inkongurenzen zwischen Hüftspacer und -kopf auszugleichen. Hierzu kann für einen besseren Verbund die Innenseite bzw. die innere Schicht auch aufgeraut sein oder aus einer vlies- oder schaumartigen Struktur bestehen. In diese porösen Strukturen können Knochenstrukturen einwachsen. Daher besteht die Innenseite beispielsweise aus Hydroxylappatit, Kalziumphosphat, Titanium oder einer anderen geeigneten Substanz, die ein teilweises Knocheneinwachsen begünstigt.

Grundsätzlich kann der Hüftspacer mit den beschriebenen Material- und Sandwichstrukturen auch in Form eines Pfanneneinsatzes konzipiert sein, also dass der Hüftspacer nicht über den Hüftkopf gestülpt wird, sondern in die Pfanne der Hüfte eingesetzt wird. Zudem kann der Hüftspacer als Dämpfungselement für Hüft-Endoprothesen in Form eines Überzuges über den Kopf oder Pfanneneinsatzes genutzt werden.

Ferner sind die in dieser Erfindung ausgeführten Materialausgestaltungen und Sandwichstrukturen auch für weitere Gelenkspacer nutzbar, die in anderen Gelenken innerhalb (Bandscheibe, Schulter, Fuß und Hand) oder außerhalb als Orthesen des menschlichen Körpers eingesetzt werden können. Sie können auch für Gelenke von Tieren eingesetzt werden, wie z.B. für Pferde. In härteren Ausführungen sind die Materialien auch als Sehnen- bzw. Bänderersatz wie z.B. als Kreuzbänder im Kniegelenk geeignet.

Außerdem eignen sich die beschriebenen Polymere für weitere Implantate des menschlichen Körpers, wie beispielsweise für Gefäße, Herzklappen und sonstige Ventile im menschlichen Körper sowie für Pumpenmembranen und -kammer in künstlichen Herzen und Herzunterstützungssystemen. Des Weiteren sind sie für ophthalmologische Implantate wie Intraokularlinsen oder künstliche Hornhäute geeignet.

Konkrete Ausgestaltungen und weitere bevorzugte Ausführungsformen der vorliegenden Erfindung werden im Folgenden anhand des Diagramms und der Figuren erläutert. Es zeigen:
- Diagramm:: Spannungs-Dehnungsverläufe,
- Fig. 1a:: Tibia und Femur eines linken Knies mit medialem (links) und lateralem (rechts) Meniskusspacer,
- Fig. 1b, c:: eine Seitenansicht eines leicht gebeugten Knies von lateral (b) und in Frontalansicht (c),
- Fig. 2:: Aufsicht auf einen linken Tibiakopf mit Randkonturen von medialem (links) und lateralem (rechts) Kniespacer,
- Fig. 3a-i:: verschiedene Ansichten eines medialen Meniskusspacers mit einer Sandwichstruktur (im Belastungszustand),
- Fig. 4 a-i:: verschiedene Ansichten eines lateralen Meniskusspacers (im Belastungszustand),

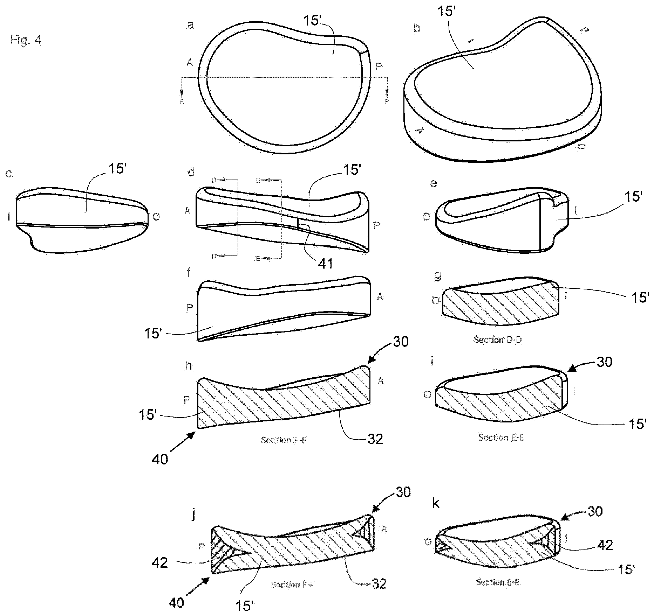

- Fig. 4 j-l:: verschiedene Ansichten des lateralen Meniskusspacers mit einem weichen Rand,

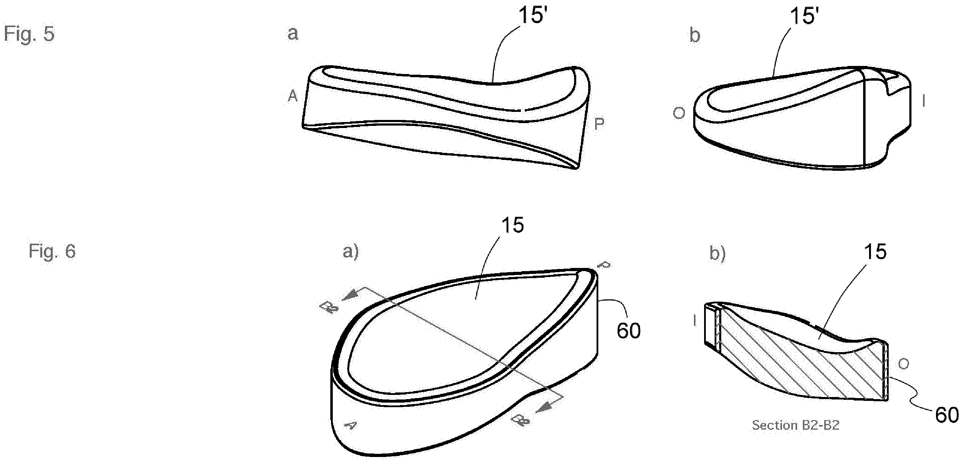

- Fig. 5 a, b:: einen lateralen Meniskusspacer mit angestellter Randfläche,

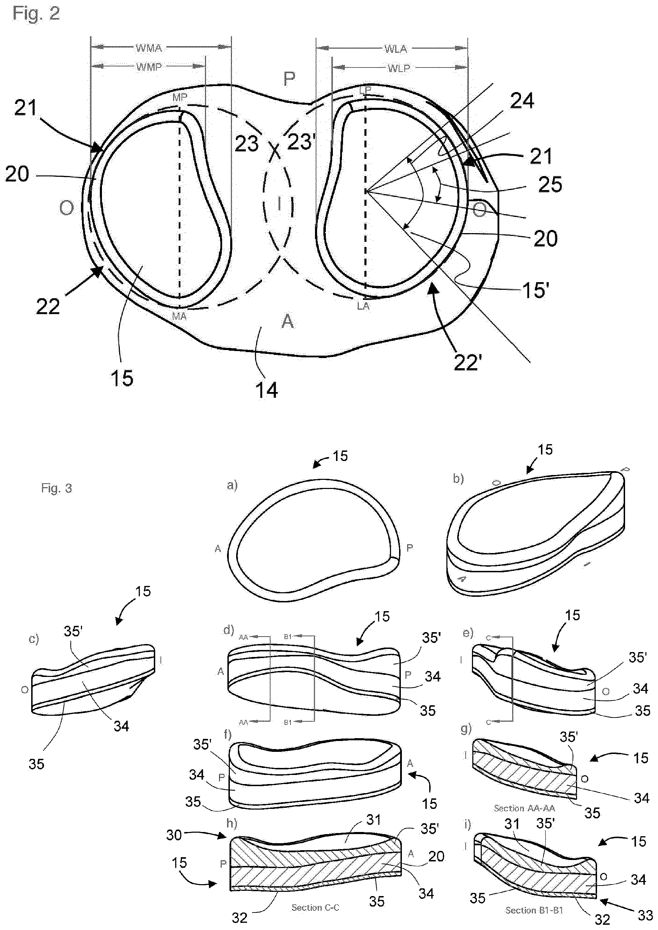

- Fig. 6 a, b:: einen medialen Spacer mit Umrandung aus vliesartiger Struktur (3D-Ansicht (a) und Schnittdarstellung (b) entsprechend Section B1-B1 aus Fig. 3),

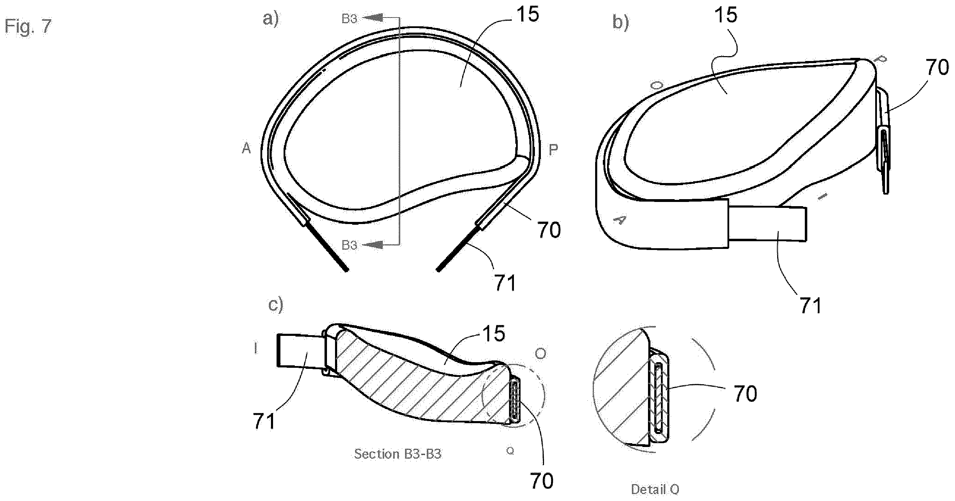

- Fig. 7 a-c:: einen medialen Spacer mit einem flachen Schlauch mit einem darin befindlichen Fixierungsband,

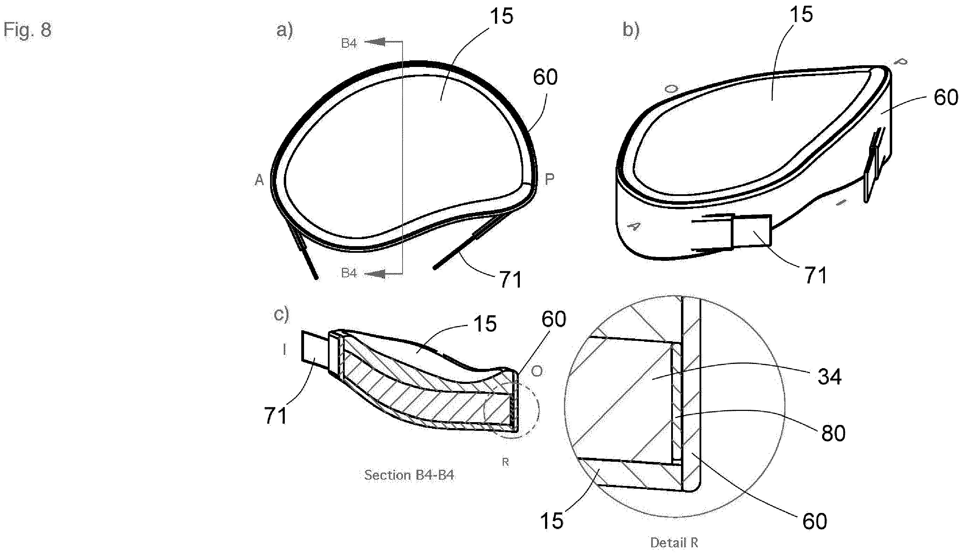

- Fig. 8 a-c:: einen medialen Spacer mit Sandwichstruktur,

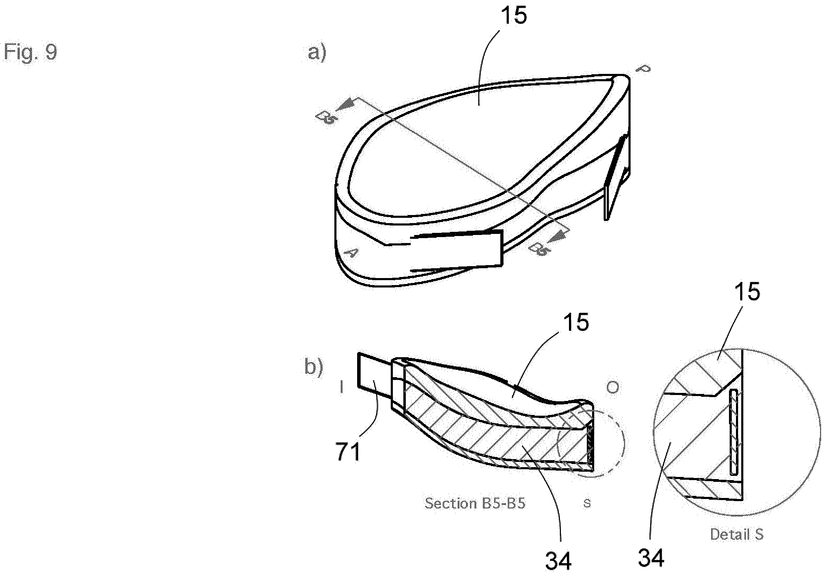

- Fig. 9 a, b:: einen medialen Spacer mit Sandwichstruktur,

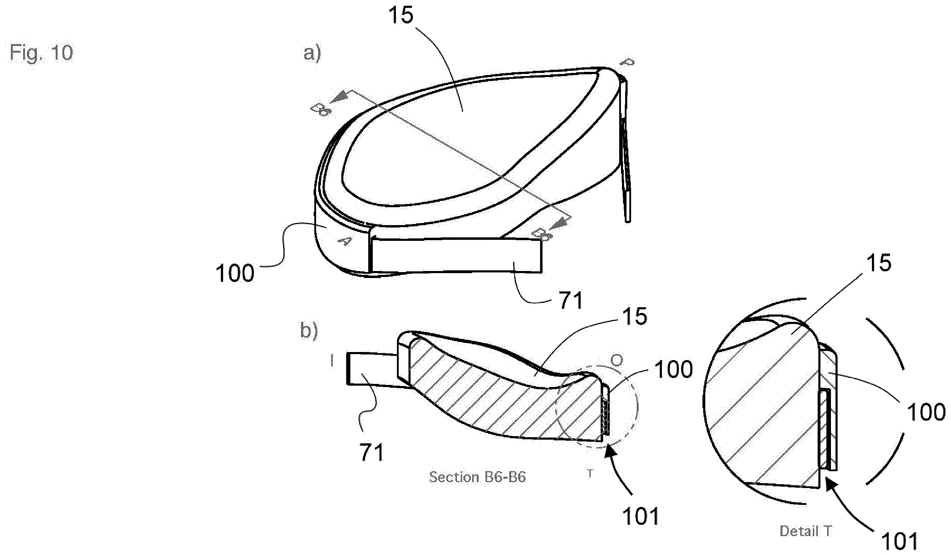

- Fig. 10 a, b:: einen medialen Spacer mit einer Profilschiene,

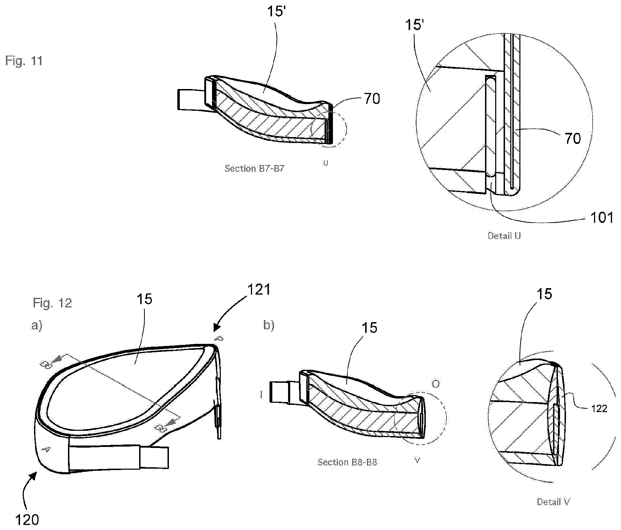

- Fig. 11:: einen medialen Spacer mit Sandwichstruktur und einer offenen Nut,
- Fig. 12 a, b:: einen medialen Spacer mit Sandwichstruktur mit einer lösbaren Profilschiene,

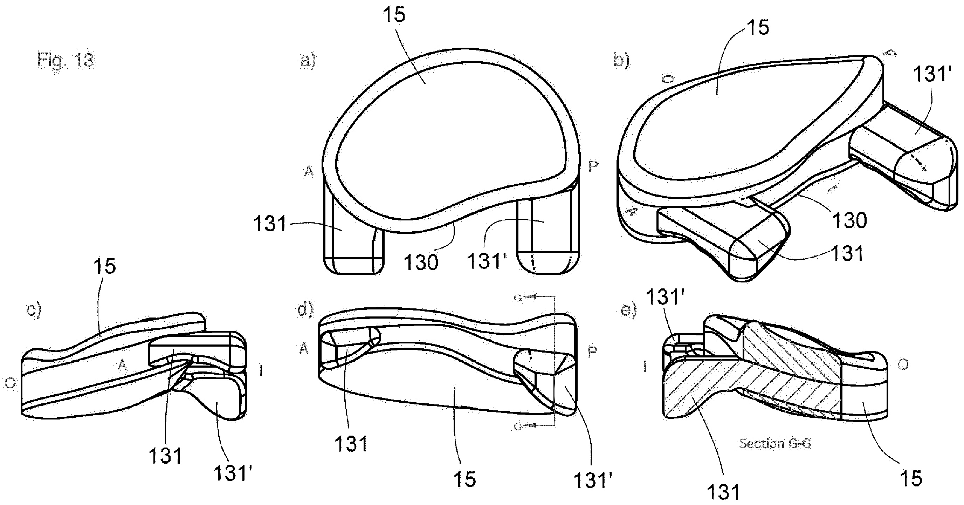

- Fig. 13 a-e:: einen medialen Spacer mit Sandwichstruktur mit Auskragungen,

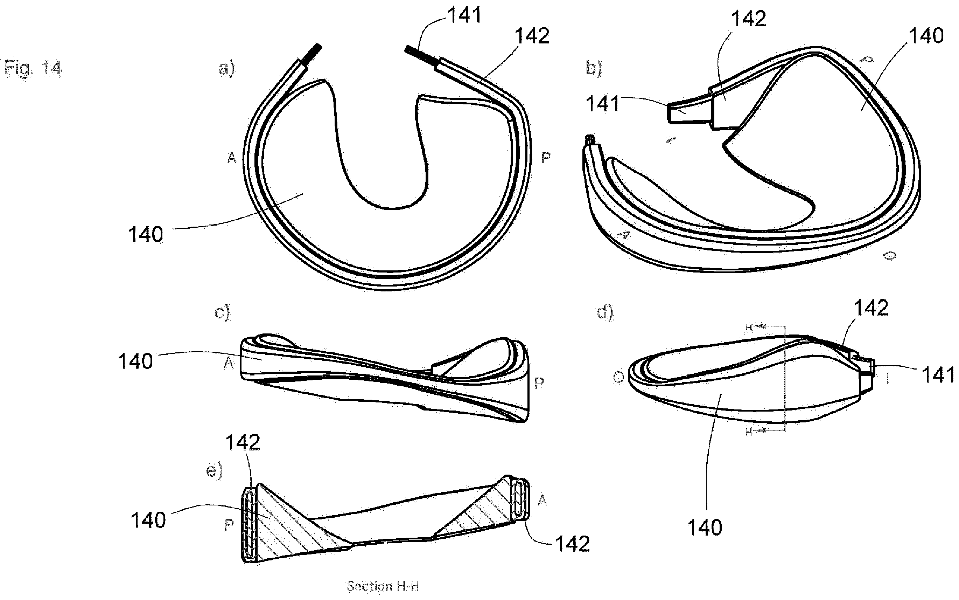

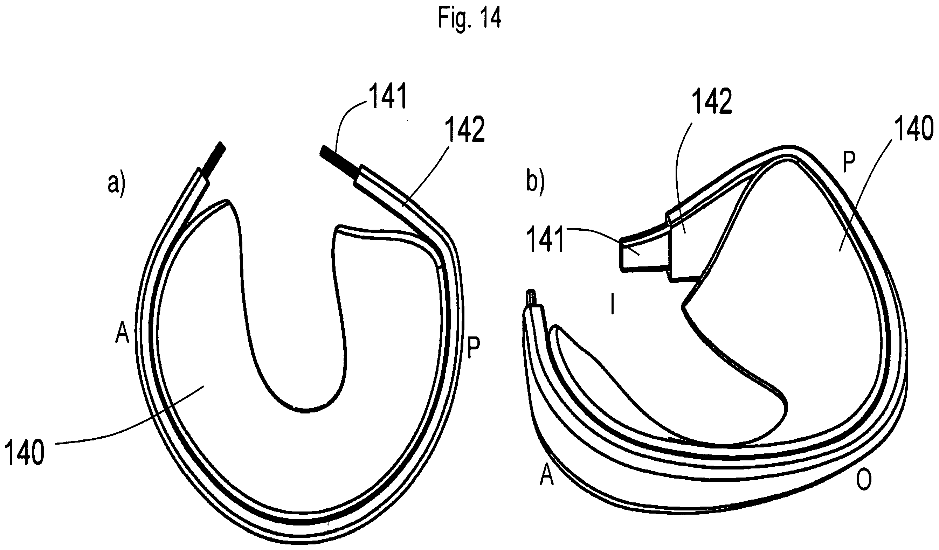

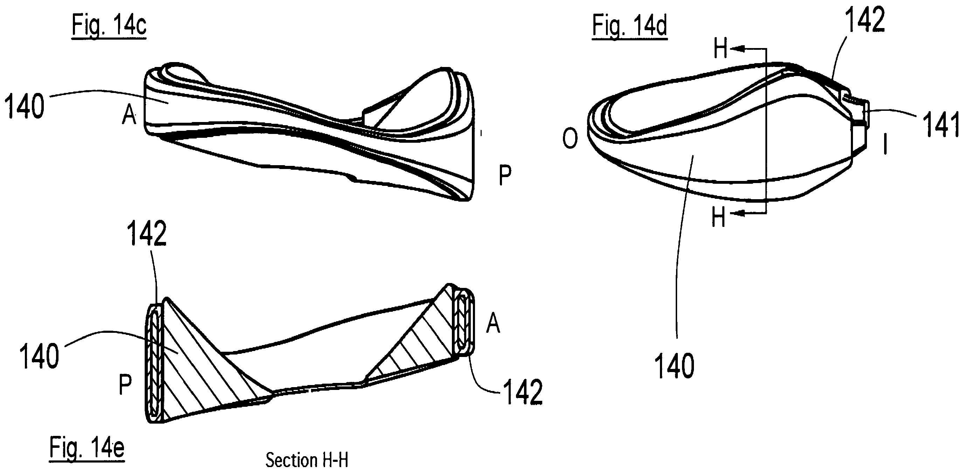

- Fig. 14 a-e:: ein laterales Meniskusimplantat mit einem Schlauch und einem darin angeordnetem befindlichen Fixierungsband,

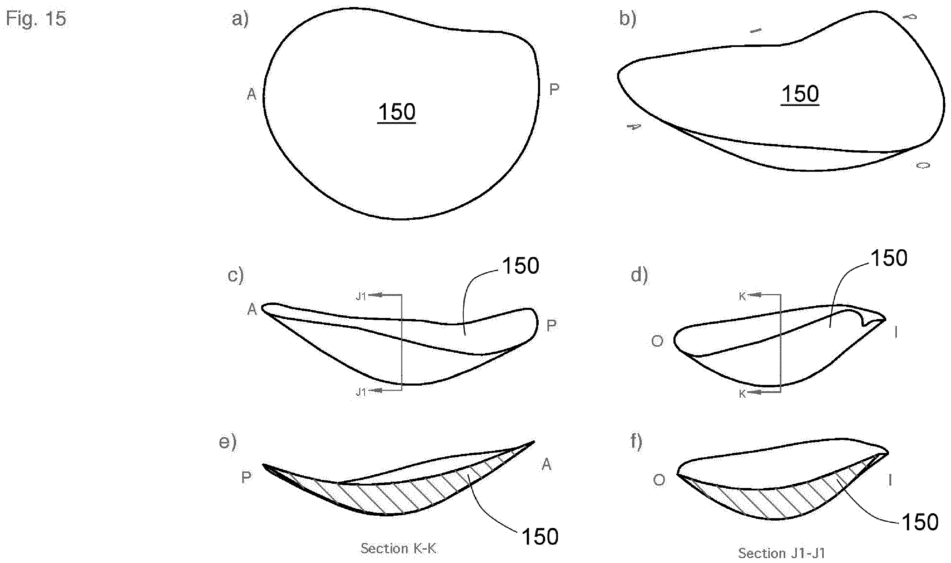

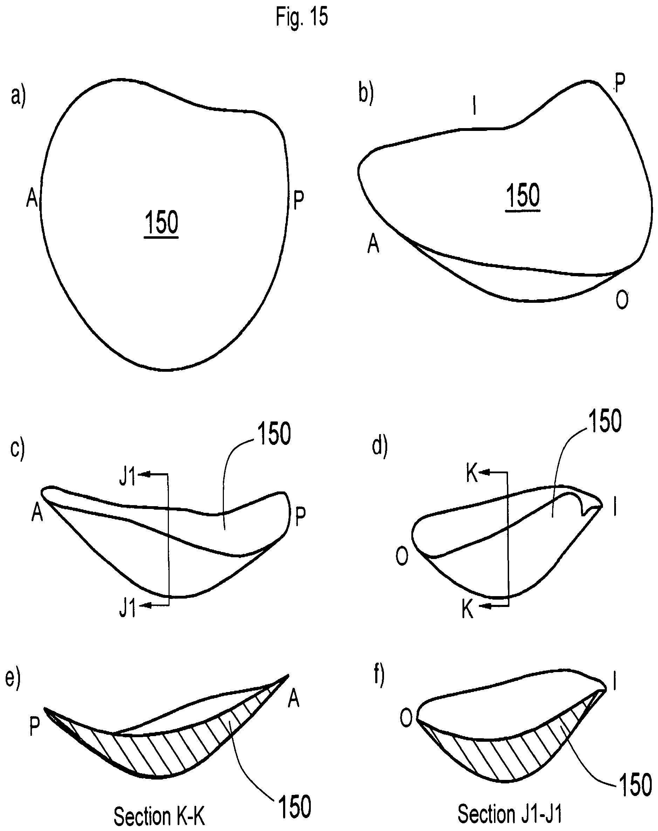

- Fig.15 a-f:: einen lateralen (femuralen) Gelenkflächenspacer,

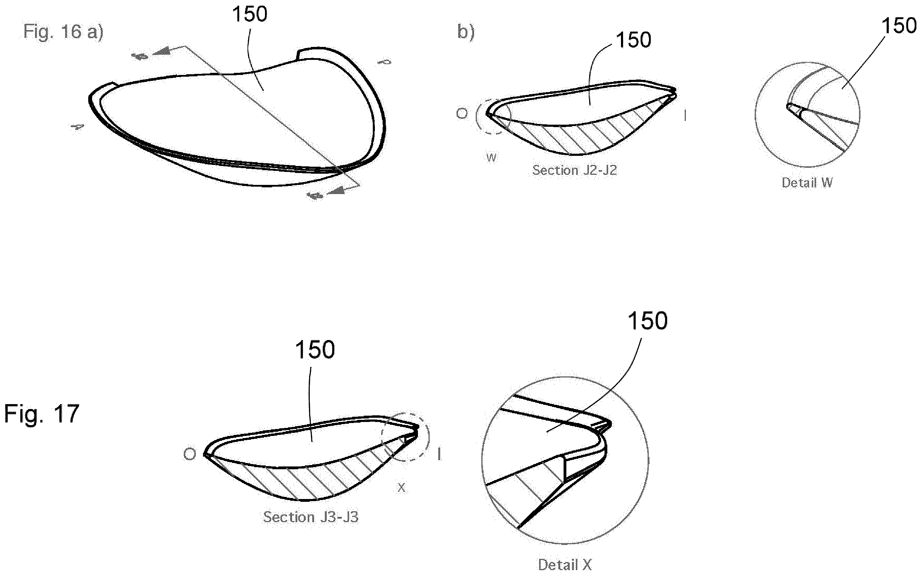

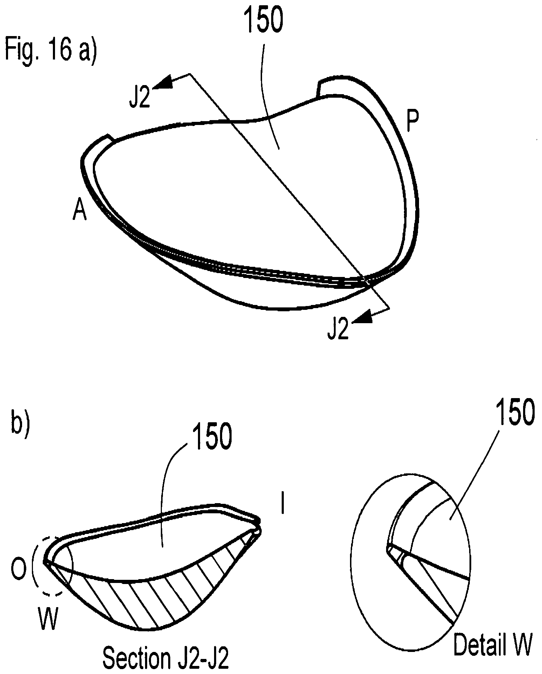

- Fig. 16 a, b:: einen lateralen (femuralen) Gelenkflächenspacer,

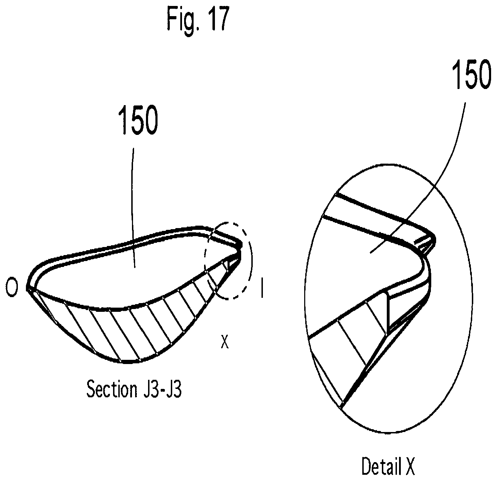

- Fig. 17:: eine Schnittdarstellung eines lateralen Gelenkflächenspacers,

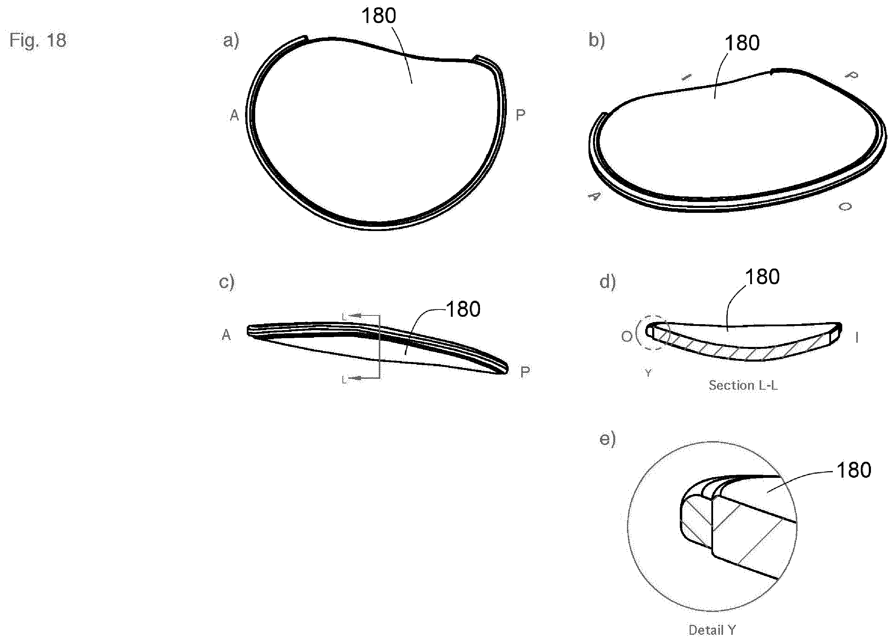

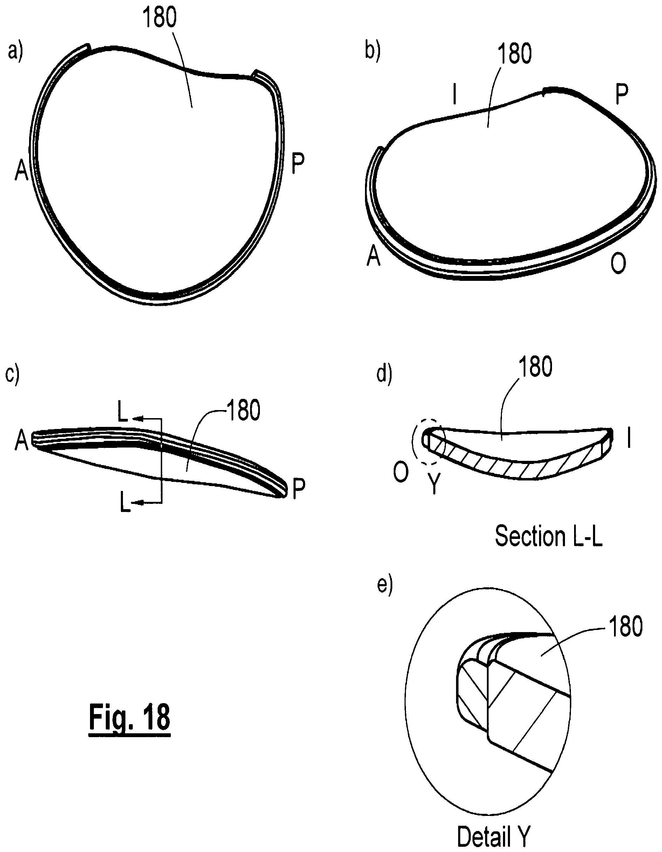

- Fig. 18 a-e:: einen lateralen (tibialen) Gelenkflächenspacer,

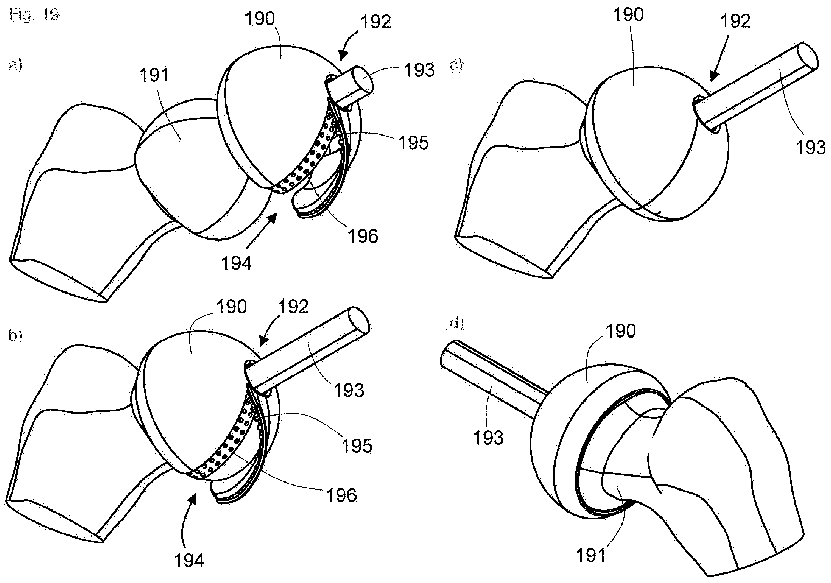

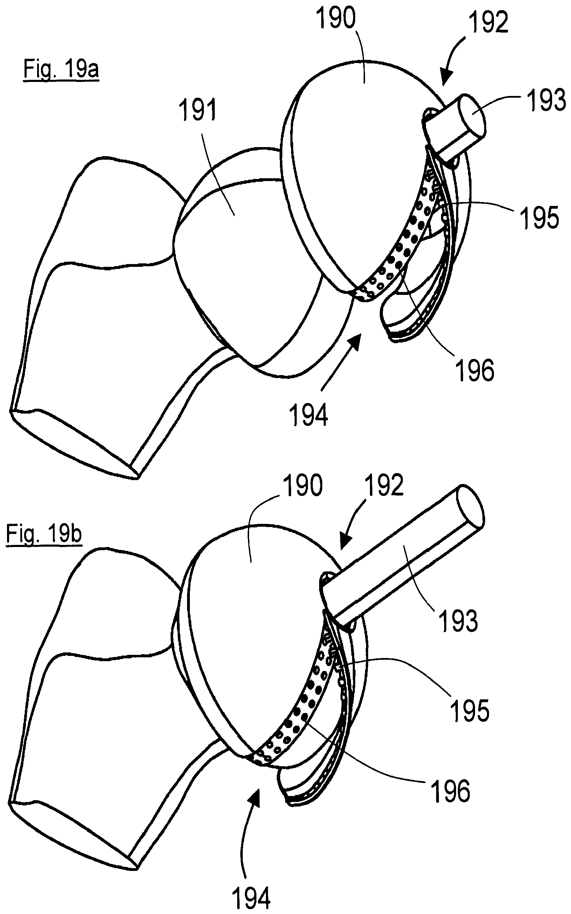

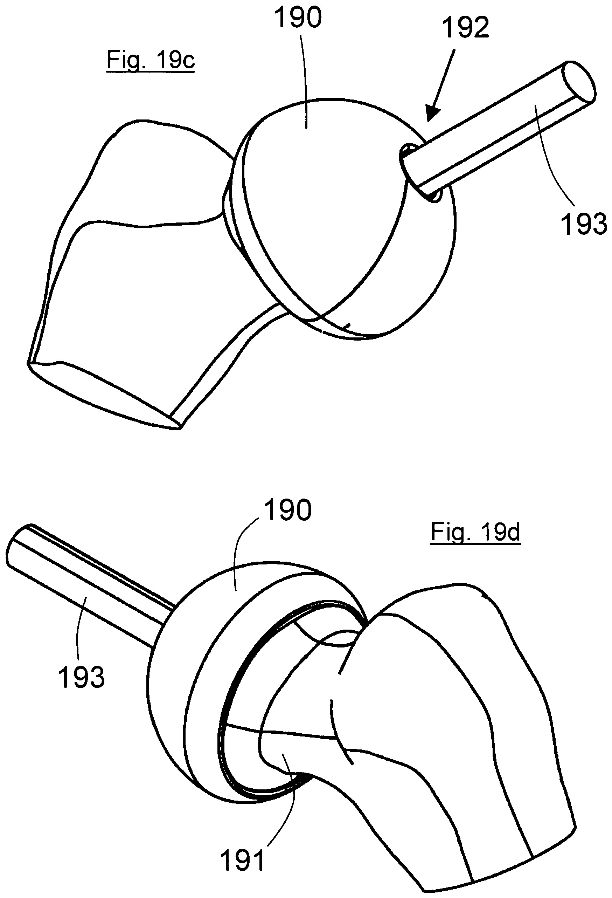

- Fig. 19 a-d:: einen Hüftspacer (a-b geöffnet, c-d geschlossen) über einem Hüftkopf mit einem Hüftkopfband,

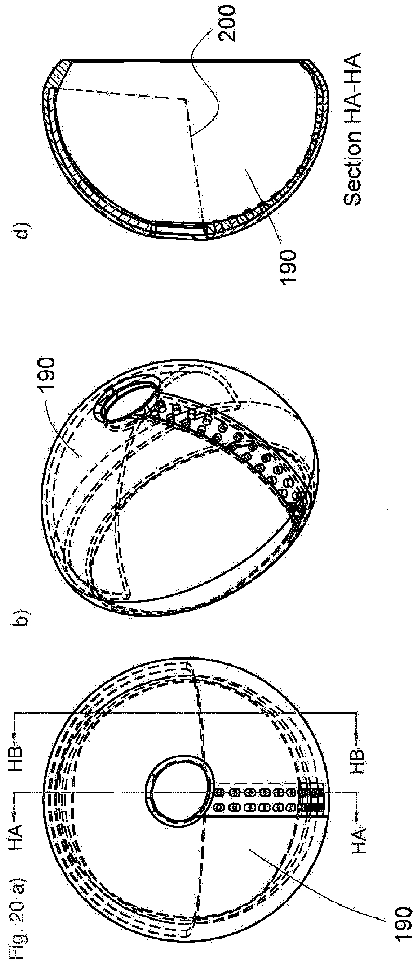

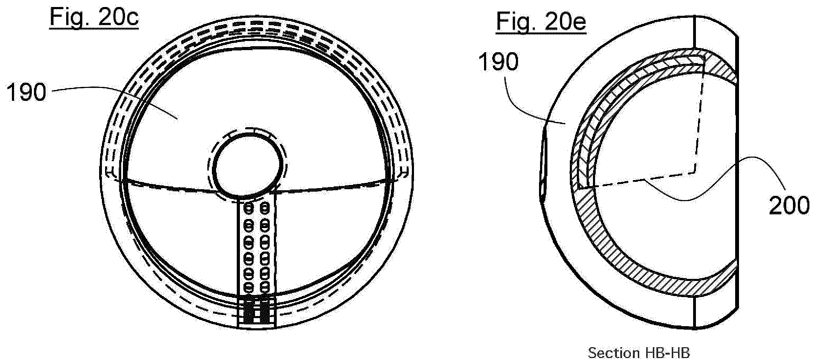

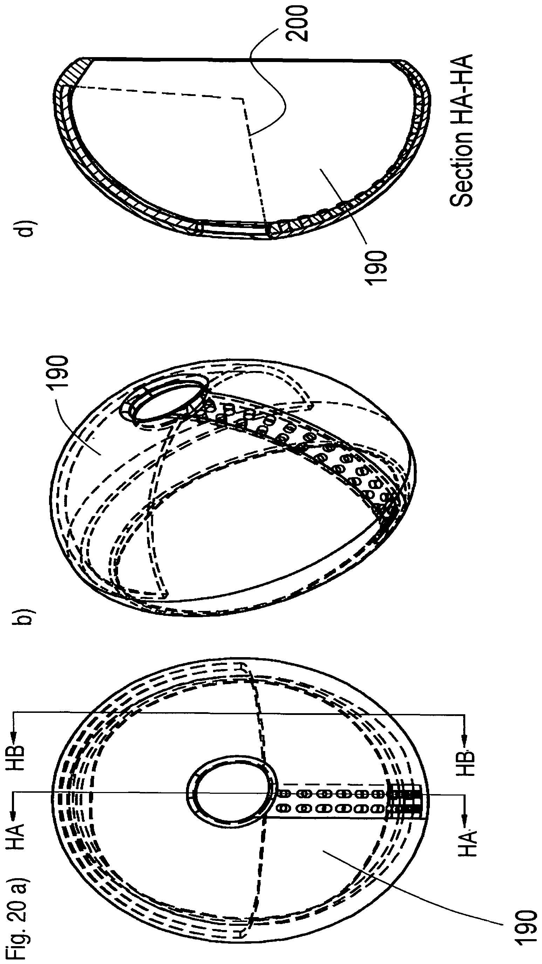

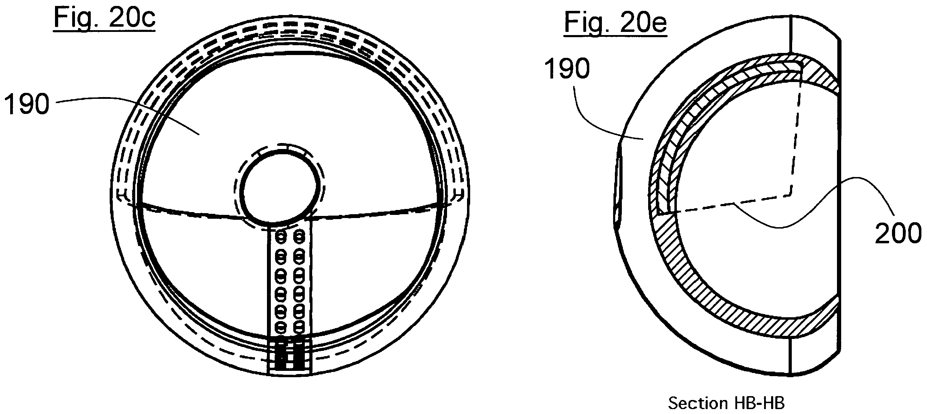

- Fig. 20 a-e:: einen Hüftspacer und

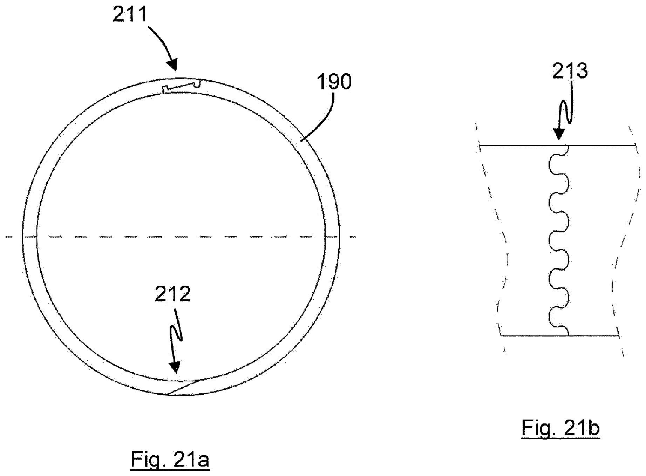

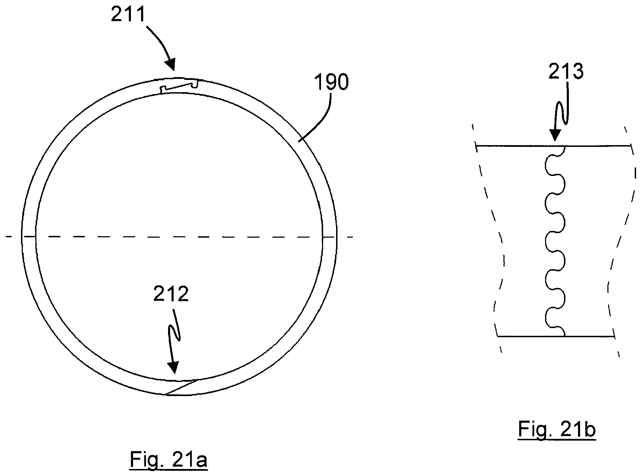

- Fig. 21 a-b:: einen Querschnitt durch den Hüftspacer mit
einer schrägen Hakenblattung (a - oben),
einem schrägen Schnitt (a - unten) und
einer Aufsicht auf eine Verzahnung in Puzzleform (b) der aufgetrennten Verbindung des Hüftspacers.

Die im Folgenden benutzten Abkürzungen bedeuten folgendes:
- a: anterior
- p: posterior
- I: innere zur Kniemitte gelegene Seite eines Kniespacers
- O: äußere Seite eines Kniespacers

In Diagramm 1 ist der charakteristische Bereich der Zug-Spannungs-/Dehnungsverläufe der bevorzugten Materialien dargestellt. Es zeigt insbesondere die 50%-Zugspannungswerte für die Materialien im Shore-A-Härte Bereich 20-77, die größer 3,8 N/mm², vorzugsweise größer 4,6 N/mm² und besonders bevorzugt größer 6 N/mm² sein sollen. Das Diagramm legt zudem einen Spannungsverlauf mit einem Fließverhalten dar, welches vermieden werden sollte, es sei denn, die Fließgrenze würde sehr hohe Werte größer als 10N/mm² erreichen. Die S-Kurve ("S-Verlauf") verdeutlicht, dass ein geeignetes Material mit ausreichender 50%-Zugspannung sehr viel niedrigere Elastizitätsmodule und Zugspannungswerte bei Dehnungen unter 50% aufweisen kann wie in den anderen gezeigten Verläufen.

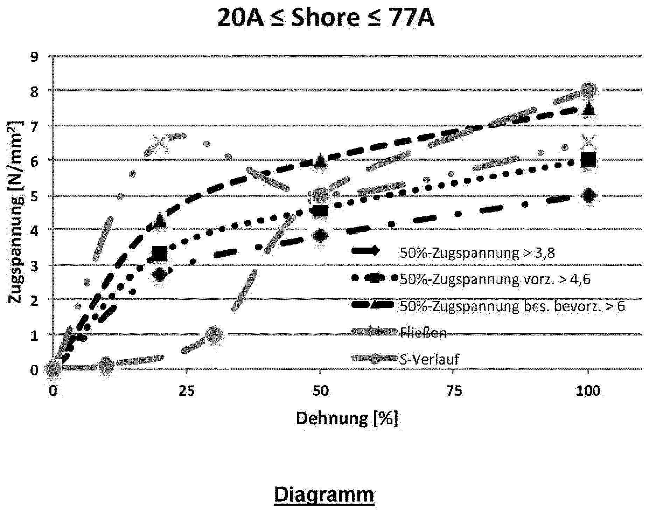

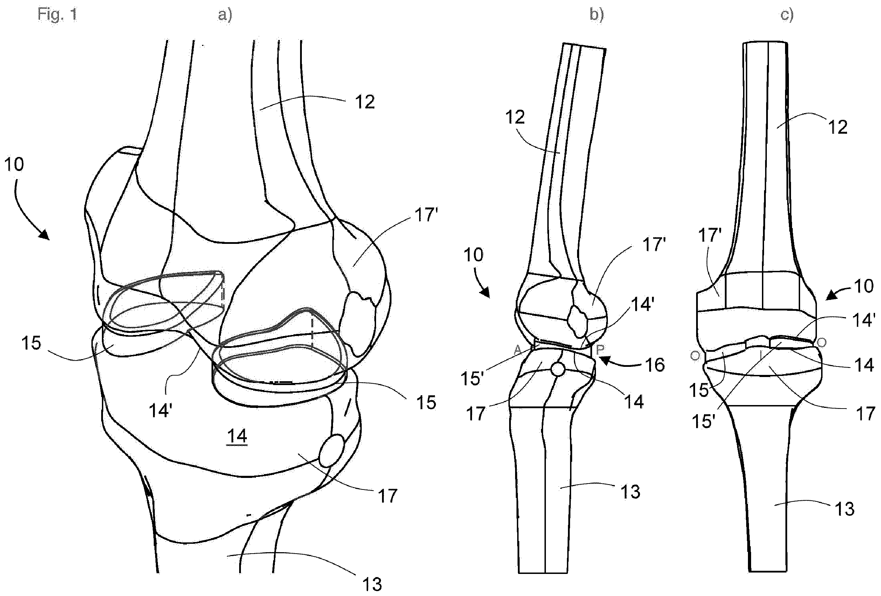

Die Fig. 1a-c zeigen unterschiedliche perspektivische Darstellungen eines (linken) Kniegelenkes 10 sowie Teilbereiche eines Oberschenkels 12 (Femur) und eines Unterschenkels 13 (Tibia). Zwischen den Gelenkflächen 14, 14' sind Meniskusspacer 15, 15' angeordnet, die den abgeriebenen und beschädigten (natürlichen) Meniskus ersetzen. Zur Materialschonung des Meniskusspacers 15, 15' müssen die zu übertragenden Kräfte auf eine möglichst große lastaufnehmende Fläche verteilt werden. Die Form, insbesondere die auf das Tibiaplateau 14 projizierte Randkontur eines Kniespacers 15, 15' spielt daher eine zentrale Rolle. Die höchsten Belastungen treten bei nahezu voll gestreckter Beinstellung auf, wie es in Fig. 1b angedeutet ist. In dieser leicht gebeugten Stellung ist aufgrund der im Verhältnis zum Tibiaplateau 14 nach posterior ansteigenden Femurkondyle und des sich dadurch vergrößernden Spaltes 16 die Beanspruchung in der anterioren Hälfte eines Kniespacer 15, 15' am höchsten. Zudem verschmälert sich die Auflagefläche des Tibiaplateaus 14 von anterior nach posterior durch eine Einmuldung (der Area intercondylaris posterior). Die posteriore Hälfte der Auflagefläche dient besonders zum Abrollen der Kondylen 17, 17' in stärker gebeugten Stellungen, in denen geringere Belastungen auftreten.

Fig. 2 zeigt, dass die Randkontur 20 und damit lasttragende Umrandung des Kniespacers 15, 15' ähnlich wie ein Fußabdruck anterior breiter und posterior schmaler ausgebildet ist. Die anteriore Hälfte ist bei der dargestellten konkreten Ausgestaltung um 10-25% breiter als die posteriore Hälfte. Die längs der anterior-posterioren Achse MA-MP, LA-LP jeweils äußere Seite der Randkontur des medialen sowie auch des lateralen Meniskusspacers ist dabei im Wesentlichen kreisbogenförmig ausgestaltet (Pfeil 21). Hierbei ist die äußere Seite der Randkontur des lateralen Meniskus-spacers 15' einem Kreisbogen stärker angenähert als die äußere Randkontur des medialen Meniskusspacers 15 (vgl. Pfeile 22, 22'). Die dem Kreisbogen entgegengesetzte innere Seite der Randkontur 23, 23' ist vom breiteren zum schmaleren Teil der Auflagefläche konkav ausgeformt. Die so gebildete Randkontur 21 des medialen als auch lateralen Meniskusspacers 15, 15' passt sich der Auflagefläche des tibialen Plateaus 14 optimal an und verteilt damit die Belastung auf eine maximal große Fläche. Zudem wird die Gefahr von Luxationen oder Verklemmungen verringert. Um das Luxationsrisiko weiter zu vermeiden bzw. zu verringern, ist der Meniskusspacer 15' anterior und posterior dicker ausgestaltet als im zentralen Bereich des Meniskus-spacers 15' und zumindest in einem Bereich am äußeren Rand gleich dick oder bevorzugt dünner als der zentrale Bereich des Meniskusspacers, wobei sich der Bereich vom Mittelpunkt der kreisförmigen Randkontur aus gesehen um einen Winkel 24 von 90°, vorzugsweise um einen Winkel 25 von 45°, erstreckt.

Die tibiale Fläche des Meniskusspacers 15, 15' wird vorzugsweise individuell der Topografie des Tibiaplateaus des Patienten angepasst. Alternativ wird vorzugsweise die tibiale und auch femurale Fläche des Knies - des Knochens und bevorzugt des Knorpelbelages - aus einem statistischen 3D-Formmodell einer Patientengruppe gemäß populationsspezifischer Eigenschaften generiert. Ein Formmodell gibt die Durchschnittsform einer größeren Anzahl einer Gruppe mit ähnlichen Formen an, mit der auch die Formvariation dieser Gruppe aufgezeigt werden kann. Hierbei können die Patientengruppen gemäß der Klassifikation nach klinischen Schweregraden der Arthrose eingeteilt werden, zudem kann hierbei auch nach Geschlecht oder Alters-/Gewichtsgruppen oder weiteren Kriterien wie ethnische Herkunft gemittelt werden. Typischerweise werden die Knieformen eines Individuums aus CT oder MRT Aufnahmen erzeugt. Bei der Generierung des 3D-Formmodells sollte die Fehlstellung der jeweils zu der Gruppe hinzugezogenen Knieformen zu einer möglichst physiologischen Beinstellung korrigiert sein. In der Praxis wird dies jedoch von den individuellen Begebenheiten des Patienten abhängen, hierbei insbesondere von dessen Bandapparat bzw. ob dieser "locker" bzw. "ausgeleiert" oder "zusammengeschrumpft" ist. Daraus ergibt sich beispielsweise, dass ein Patient mit einem lockeren Bandapparat leichter zu einer korrekten Beinstellung durch Einsetzen eines Kniespacers übergeführt werden kann gegenüber einem Patienten mit einem geschrumpften Bandapparat, wo der für den Kniespacer einzusetzende Spalt für eine normale Beinstellung zu klein ist. Letztere Kriterien sind daher auch bei der Generierung der 3D-Formmodelle in den einzuteilenden Gruppen zu berücksichtigen und zwar beispielsweise durch vorgegebene Fehlstellungsgrade des Beines.
Die femurale Fläche des Meniskus-Spacers 15, 15' ergibt sich aus Abgüssen des Femurs unter Winkel- bzw. Beugestellungen (0-40°) des Femurs, in denen die Kraftmaxima auftreten. Vorzugsweise liegt der Abformungsbereich bei Beugungswinkeln zwischen 6° und 28°, bevorzugt wiederum im Bereich des Mittelwerts dieses Winkelbereiches. Die Abformung der femuralen Seite ergibt sich jedoch vorzugsweise aus mehreren Stellungen des Femurs bzw. durch Drehen des Femurs innerhalb des bevorzugten Winkelbereiches, wobei hierbei nicht der gesamte genannte Winkelbereich abgeformt werden muss. Durch diese Formgebung wird das Luxationsrisiko enorm verringert. Die Abformung der femoralen Seite kann patientenindividuell oder gemäß den zuvor beschriebenen statistischen 3D-Formmodellen erfolgen. Aus letzteren lassen sich dann vorkonfektionierte bzw. vorgefertigte Spacer ableiten, die zum einen zu den jeweiligen ausgeprägten 3D-Formmodellen gehören und zum anderen in unterschiedlichen Größen, aber auch Dicken, bereitgestellt werden. Die Einteilung nach Dicken erfolgt, weil dadurch unphysiologische Abwinklungen des Beines (Valgus/Varus) bzw. je nach Patient und dessen Gewicht und Abnutzungsgrad des Knorpelbelages des Knies unterschiedliche Gelenkspalte ausgeglichen werden können.

Für die Auswahl der passenden Dicke des Kniespacers und der Kniespacerform wird vorzugsweise folgendes Verfahren vorgeschlagen. Mit einem oder mehreren bildgebenden Verfahren wird das Bein des Patienten erstens stehend unter Last, wo Tibia und Femur in Kontakt stehen und das Bein eine Fehlstellung aufweist, und zweitens begradigt, also mit korrekter Beinstellung, aufgenommen, hierbei vorzugsweise aus Vorder- und Seitenansicht. Aus den zweiten Aufnahmen kann der sich ergebende Spalt zwischen Femur und Tibia für die Auswahl der Dicke herangezogen werden. Vorzugsweise wird jedoch die Differenz des Abstandes von Femur und Tibia zwischen den beiden Aufnahmen für die Auswahl der geeigneten Dicke des Kniespacers herangezogen. Vorteilhaft ist, wenn hierbei auf einfache und kostengünstigere 2D-Aufnahmen wie z.B. Röntgenaufnahmen zurückgegriffen werden kann. Ferner ist zudem vorteilhaft, wenn aus den 2D-Aufnahmen anhand charakteristischer Landmarken oder sonstiger Ausprägungen auf das für den individuellen Patienten repräsentierende statistische 3D-Formmodell und damit auf die richtige Form des Kniespacers geschlossen und ein vorkonfektionierter bzw. vorgefertigter Kniespacer ausgewählt werden kann. Aus einer weiteren seitlichen Aufnahme unter einer der zuvor genannten vorzugsweise maximalen Beugestellungen des Beines kann die relative Position des Femurs zur Tibia verifiziert werden, welches für die Abformung und damit Formgebung des Kniespacers wichtig ist.

Die Fig. 3 a-i zeigen den medialen Meniskusspacer 15 und die Fig. 4 a-i den lateralen Meniskusspacer 15'. Die Übergänge zwischen den durch die Randkonturen 20 gebildeten Randflächen und den femuralen Flächen 31 des Meniskusspacer 15, 15' sind gerundet (Pfeil 30), da der Femur 12 über die femurale Fläche 31 gleitet und abrollt. Die Übergänge zwischen den Randflächen und den tibialen Flächen 32 des Meniskusspacer können gerundet (Fig. 4, Pfeil 40) oder auch nicht gerundet sein (Fig. 3, Pfeil 33). Letzteres insbesondere dann, wenn der Meniskusspacer 15, 15' auf dem Tibiaplateau 14 fixiert wird und es folglich zu keiner Relativbewegung zwischen beiden kommen kann. Vorzugsweise soll der Meniskusspacer 15, 15' jedoch auf dem Tibiaplateau 14 beweglich sein.

Durch die zuvor beschriebene Generierung der tibialen sowie femuralen Fläche 31, 32 des Meniskusspacers variiert die Höhe der seitlichen Randfläche entlang des Umfangs. Die Randflächen sind beim medialen wie lateralen Meniskusspacer 15, 15' posterior und anterior im Durchschnitt höher als an den Seitenflächen, wobei sie insbesondere posterior am Übergang zur inneren Randfläche am höchsten sind. Ferner ist insbesondere die innere Randfläche des medialen Meniskusspacer 15 in der anterioren Hälfte in Nähe des Übergangs zur posterioren Hälfte (zwischen Schnitten g und i in Fig. 3) am dünnsten. Das Tibiaplateau 14 ist in diesem Bereich in medial-lateraler Richtung zur Ebene des Plateaus insbesondere zum inneren Rand hin geneigt bzw. "abschüssig" (s. Fig. 1c und Schnittdarstellung Fig. 3i). Je dicker der Spacer hier ist, je höher ist das Risiko, dass durch die vertikale Belastungsrichtung der Spacer seitlich luxieren kann. Dagegen ist beim lateralen Meniskusspacer 15' die innere Randfläche höher bzw. je nach Abnutzung bei Valgus-Stellung und Abflachung des Tibiaplateaus 14 etwa gleich hoch wie die äußere Randfläche. Das laterale Tibiaplateau 14 ist konkav geformt und im Wesentlichen senkrecht zur Beinachse (also nicht abschüssig) angeordnet, womit der resultierende Kraftvektor weitestgehend mit der vertikalen Beinstellung koaxial ist, so dass das seitliche Luxationsrisiko des lateralen Meniskusspacer geringer ausfällt.

In Fig. 4d und i ist zudem die dünnste Stelle bzw. geringste Höhe 41 des äußeren Randes gekennzeichnet, wobei gleichzeitig die Dicke an dieser Stelle gleich oder vorzugsweise geringer ist als im zentralen Bereich.

Die tibiale Fläche 32 des Meniskusspacers 15, 15' ist im Wesentlichen konvex und die femurale Fläche 31 konkav ausgebildet. Die Krümmung der femuralen Fläche 31 des Meniskusspacers 15, 15' ist bevorzugt größer als die der Femurkondyle, so dass der Meniskusspacer 15, 15' zunächst an ihren besonders elastischen Rändern belastet wird und damit eine hohe Dämpfung gegeben ist.

Entgegen den Darstellungen in Fig. 3 und Fig. 4 muss die Randfläche des Meniskusspacers nicht in "vertikaler" Richtung verlaufen, sondern kann auch in der durch die Beinachse und die anterior-posteriore Richtung gebildeten Ebene

(Sagittalebene) angestellt sein, wie es in Fig. 5 gezeigt ist. Ferner kann die Randfläche aus fertigungstechnischer Sicht auch so angestellt sein, dass eine Entformung des Spacers aus dem Fertigungswerkzeug erleichtert wird.

Insgesamt folgt durch diese Formgebung der Meniskusspacer 15, 15' der natürlichen Kniebewegung, passt sich aufgrund der nachfolgend weiter ausgeführten hohen Komplianz des Materials der jeweiligen Beugung des Gelenks an und ist selbstzentrierend. Bei normalem Bewegungsablauf können so Luxationen, wie auch Bewegungseinschränkungen oder ein Klemmen des Spacers mit zu hoher Beanspruchung und möglicher Schädigung, weitestgehend vermieden werden.

Es wurde bereits ausführlich erläutert, dass der Gelenkspacer, der nach einer konkreten Ausgestaltung der vorlegenden Erfindung eine Sandwichstruktur aufweist, ein progressives Druck-Stauchungsverhalten zeigt. Fig. 3 zeigt beispielhaft einen Spacer mit einer Sandwichstruktur mit einer Kernschicht 34 und zwei Deckschichten 35, 35'. Die tibiale Deckschicht 35 besitzt nach einer konkreten Ausgestaltung eine gleichmäßige Dicke. Die Kernschicht 34 schließt sich mit nahezu konstanter Dicke dieser Deckschicht 35 vorzugsweise vollflächig an. Die Dicke der Kernschicht 34 des medialen Meniskusspacers 15 kann jedoch insbesondere zwischen der Linie MA-MP (Fig. 2, Linie LA-LP beim lateralen Meniskusspacer) und dem inneren Rand abnehmen (vgl. Fig. 3 g), um das seitliche Luxationsrisiko bei zur Mitte des Knies ansteigendem Tibiaplateau klein zu halten. Außerdem muss sich die Kernschicht 34 nicht vollständig bis zum Rand des Spacers ausdehnen. Die tibiale Deckschicht hat eine Dicke bis etwa 3 mm, vorzugsweise zwischen etwa 0,2 und 2 mm. Die Kernschicht 34 hat in Abhängigkeit von der Gesamtdicke des Meniskusspacers 15, 15' bzw. des Kniespaltes eine Dicke vorzugsweise im Bereich von etwa 3-10mm. Auf die Kernschicht 34 folgt proximal die femurale Deckschicht 35' mit ungleichförmiger Dickenverteilung, die zentral relativ dünn und zum Rand hin eine zunehmende Dicke aufweist. Die weichen Deckschichten 35 und 35' mit ihrem dickeren Randbereich können Stoßbelastungen außerordentlich gut dämpfen. Die hohe Nachgiebigkeit der Sandwichstruktur sorgt dafür, dass sich der Meniskusspacer 15, 15' der Kniebewegung während Beugung und Streckung sehr gut anpassen kann. Dadurch wird das Luxationsrisiko weiter reduziert.

In einer weiteren Ausgestaltung kann die Kernschicht 34 des Meniskusspacers 15, 15' zum äußeren, kreisbogenförmigen Rand hin verdickt sein, beispielsweise dann, wenn der Spacer, wie es weiter unten bzgl. Fig. 9b beschrieben ist, mit einem zusätzlichen umlaufenden Band auf dem Tibiaplateau 14 fixiert wird. Diese Verdickung bildet damit eine in der Aufsicht zusätzliche C-förmige Verstärkung der Kernschicht entlang ihres äußeren Randes.

Die äußeren weichen Deckschichten 35, 35' der Sandwichstruktur eines Gelenkspacers 15, 15' bestehen ferner vorzugsweise aus einem besonders hydrophilen und/oder reibungsarmen bzw. abriebfesten Material oder sind zusätzlich mit einer dünnen Schicht aus einem solchen Material beschichtet. In einer weiteren alternativen Ausgestaltung kann eine Deckschicht 35, 35' auch eine raue Oberfläche haben oder mit einer Schicht aus z. B. Hydroxylappatit verbunden sein, damit sie mit den umliegenden Gewebestrukturen oder dem Knochen einer Seite des Gelenkes verwachsen kann. Die zuvor beschriebene Sandwichstruktur bzw. deren einzelne Schichten bestehen vorzugsweise aus homogenen Materialen. Alle oder nur einzelne Schichten, vorzugsweise die äußeren Deckschichten können jedoch auch aus den beschriebenen porösen Strukturen bestehen. Ferner kann der Gelenkspacer auch weitere Zwischenschichten haben, um z. B. eine geringere Abstufung der Härteunterschiede zu erzielen oder auch einen größeren Bereich an Härten aufzubringen. Außerdem könnten die einzelnen Schichten nicht flächig, sondern nur an ihren Rändern miteinander verbunden sein oder ggf. auch untereinander verschiebbar sein.

Die Fig. 4j-l zeigen Ausgestaltungen des Meniskusspacers, wo nur der Randbereich in einer Sandwichstruktur ausgebildet ist und in den Randbereich des aus einem härteren Material bestehenden Spacers zumindest teilweise über den Umfang ein keilförmiger Ring aus einem weicheren Material eingelassen ist. Die Randbereiche können auch anders ausgestaltet sein als in den Figuren dargestellt, beispielsweise kann das weiche Material randseitig auf dem härteren aufgelagert sein, oder der Rand besteht über die ganze Dicke aus weichem Material.

Um das Risiko einer Luxation auch für sportlichere bzw. mobilere Patienten zusätzlich zu reduzieren, ist nach einer konkreten Ausgestaltung vorgesehen, dass die Randfläche des Meniskusspacers 15, 15' entlang ihres gesamten Umfangs mit einer porösen Vlies-Schicht 60 aus feinfibrillärer Struktur (Wirrvlies) bedeckt ist (Fig. 6), in die umliegende Zell- bzw. Gewebestrukturen einwachsen, ohne verhärtendes narbiges Bindegewebe zu bilden. Der Meniskusspacer 15, 15' kann so insbesondere mit der Kapsel des Knies verwachsen und zur Positionierung und Luxationsreduzierung im Knie beitragen. Nach einer weiteren Ausführungsform ist vorgesehen, dass die feinfibrilläre Randschicht 60 jeweils nur am distalen und proximalen Rand mit dem Kniespacer verbunden ist (Fig. 12b, Pfeile 120, 121). Vorteilhaft ist hierbei, dass es zu kleinen Relativbewegungen und damit zu einem Bewegungsausgleich zwischen Meniskusspacer 15, 15' und angewachsener feinfibrillärer Randschicht kommt und damit die Positionierung des Meniskusspacers weiter unterstützt wird. Hierzu ist nach einer konkreten Ausführungsform vorgesehen, dass um die Randfläche des Meniskusspacers ein flacher Schlauch 70 aus feinfibrillärer Struktur befestigt ist (Fig. 11, Fig. 7).

In einer alternativen Ausgestaltung ist zur Verhinderung einer Luxation vorgesehen, den Schlauch 70 lediglich an dem äußeren kreisbogenförmigen Rand zu befestigen, diesen Schlauch dabei über die Enden des Kreisbogens zum Innern des Knies zu verlängern (Fig. 7). Der Schlauch 70 hat durch diese Ausgestaltung in der Aufsicht eine C-Form. In dessen Innerem verläuft ein flexibles und gleichzeitig hochfestes Fixierungsband 71. Dieses Fixierungsband 71 kann auch an den entsprechenden Stellen aus dem Schlauch 70 bzw. der Vliesumrandung durch Öffnungen hinausgeführt sein, wenn der Schlauch um den vollen Umfang der Randfläche des Meniskusspacers 15, 15' gelegt ist (Fig. 8, Fig. 11).

Fig. 8 zeigt eine Ausführungsform, bei der das Fixierungsband 71 in einer umlaufenden Nut 80 bzw. Aussparung in der Kernschicht 34 verläuft und aus der feinfibrillären Randschicht 60 ähnlich wie zuvor beschrieben hinausgeführt wird. Das Fixierungsband 71 oder sonstige in gleicher Art und Form genutzte Verstärkungsfasern sind hierbei in der Nut 80 frei beweglich, es kann jedoch auch in der Kernschicht 34 integriert und fixiert sein, wie dies in Fig. 9 dargestellt ist. Dort ist auch die Kernschicht 34 zum äußeren Rand verdickt und verstärkt damit die Aufhängung durch das Fixierungsband 71. Alternativ kann das Fixierungsband 71 nur um die Randfläche des Meniskusspacers 15, 15' angeklebt oder andersartig befestigt sein. Die feinfibrilläre Vliesschicht ist dann jeweils am distalen und proximalen Rand mit der Randfläche befestigt.

In einer weiteren Ausführung ist vorgesehen, dass um den Kreisbogen eine im Querschnitt L-förmige Profilschiene 100 verläuft (Fig. 10), die distal bzw. zur tibialen Seite eine umlaufende Nut 101 freigibt, in die das Fixierungsband 71 eingelegt ist. Das Fixierungsband 71 könnte nach den zuvor beschriebenen Fixierungsmethoden dauerhaft im Knie verbleiben, so dass bei einer Reoperation nur der Meniskusspacer 15, 15' ausgetauscht werden muss. Der Außenbereich des L-Profils 100 ist bevorzugt mit einer feinfibrillären Struktur bedeckt. Vorzugsweise ist das Profil 100 mit der festen Kernschicht 34 des Meniskusspacers 15, 15' verbunden und besteht aus einem besonders harten Polyurethan, einem hochfesten Kunststoff oder einem Metall. Das außerhalb des eigentlichen Meniskusspacers 15, 15' liegende Fixierungsband 71 sowie die Verbindungsstelle mit dem Meniskushorn sind nach einem konkreten Ausführungsbeispiel mit der feinfibrillären Schlauchstruktur umschlossen. Die Nut 101 ist bei der Ausführungsform gemäß Fig. 11 in der Sandwichstruktur des Meniskusspacers 15, 15' integriert.

Fig. 12 zeigt eine weitere Ausführung eines Meniskusspacers 15, 15' mit einer Profilschiene 122, wobei diese sich bogenförmig oder andersartig formschlüssig in die Randfläche des Meniskusspacers 15, 15' schmiegt, jedoch nicht mit dem Spacer stoffschlüssig verbunden ist. Die Profilschiene 122 kann auch um die gesamte Randfläche des Meniskusspacers 15, 15' verlaufen. Durch diese Ausgestaltung kann der Meniskusspacer 15, 15' von der Profilschiene 122 gelöst und ebenfalls herausgehoben und durch einen anderen ausgetauscht werden. Auf die zusätzliche Profilschiene könnte bei dieser Ausgestaltung auch verzichtet werden, so dass das Fixierungsband mit einem ggf. umgebenden feinfibrillären Schlauch C-förmig um den Meniskusspacer gelegt ist.

In einer weiteren Ausgestaltung ist vorgesehen, dass ein medialer und lateraler Kniespacer 15, 15' über ein ein- oder zweistückiges Fixierungsband 71 miteinander verbunden sind. Hierbei ist jeweils das anteriore Bandende des einen Spacers mit dem posterioren Ende des jeweils anderen über Kreuz verbunden. Bei zwei- oder ggf. auch mehr als zweistückigen Fixierungsbändern 71 erfolgt eine Verbindung der Bänder vorzugsweise über Kupplungen oder Schnappverbindungen, wie beispielsweise Gürtelschnallen, Gürtelschnappschnallen, Nut- und Feder-Schnallen, über die die Bänder gleichzeitig auch in ihrer Länge geändert werden können. Die Längenänderung könnte hierbei mittels eines separaten Elementes, wie z. B. einem Gummizug oder anderer spezieller Clips bzw. (Klemm-)Schnallen wie sie bei Rucksäcken, Kofferbandstraffern oder Spangurten oder auch als Kabelbinder verwendet werden, erfolgen. Die aufgeführten Elemente können auch bei den vorherigen Ausführungen zum Einsatz kommen. Die feinfibrilläre Schlauchstruktur könnte nach erfolgter Betätigung der Kupplung über diese oder auch über die sonstigen zur Längenänderung dienenden Elemente in ähnlicher Weise wie bereits zuvor beschrieben geschoben werden.

Fig. 13 zeigt in einer anderen Ausgestaltung einen Meniskusspacer 15, 15', der an dem inneren Rand 130 und jeweils an den Enden des äußeren Randes zwei zunächst nach innen und dann nach distal gerichtete Auskragungen 131, 131' aufweist, die in die oben erwähnten Einmuldungen auf dem Tibiaplateau 14 formschlüssig greifen. Diese in Frontalansicht L-förmigen Auskragungen 131, 131' bestehen vorzugsweise aus demselben Material wie die Kernschicht 34 und sind mit dieser verbunden. Die Auskragungen 131, 131' oder Teilbereiche der Auskragungen 131, 131' können alternativ auch aus einer feinfibrillären oder anderen geeigneten Struktur bestehen, die ein Annähen der Auskragungen 131, 131' mit den Meniskushörnern ermöglichen. Sie können auch elastisch ausgelegt sein, so dass eine anterior-posteriore Bewegung des Kniespacers 15, 15' erleichtert wird. Ferner können die Auskragungen 131, 131' an ihren Enden stiftförmig gestaltet sein, dass sie in an den Einmuldungen getätigte Bohrungen reinragen und damit eine Luxation des Meniskusspacers 15,15' verhindern. Die Randfläche des Meniskusspacers 15, 15' ist hier wieder vorzugsweise mit einer feinfibrillären Schicht bedeckt.

Im Folgenden werden weitere Ausführungen des Kniespacers beschrieben. Fig. 14 zeigt das laterale Meniskusimplantat 140, welches nur den Meniskus im Knie ersetzt. Dieser ist wie der natürliche Meniskus in der Aufsicht halbmondförmig und im Querschnitt keilförmig. Das umliegende Band 141 mit schlauchförmiger Außenhülle 142 ist ähnlich wie beim Meniskusspacer ausgebildet. Mit dem Meniskusimplantat können auch die anderen zuvor beschriebenen Elemente zur Fixierung und damit Luxationsvermeidung kombiniert werden.

Fig. 15 zeigt eine bevorzugte Ausgestaltung eines lateralen "femuralen" Gelenkflächenspacer 150 für Menschen mit einer Arthrose in einem frühen Stadium, bei der der Meniskus noch in einem solchen Zustand ist, dass er nicht oder nicht vollständig entfernt werden muss. Der Gelenkflächenspacer 150 wird in die konkave Ausmuldung des Tibiaplateaus 14 gelegt, welche durch den im Knie verbleibenden Meniskus gebildet wird. Dieser Gelenkflächenspacer 150 ist tibialseitig daher zum Rand hin abgeschrägt, so dass tibiale und femurale Randkontur ineinander übergehen. Eine Randfläche wie beim Meniskusspacer existiert nicht. Die tibiale Seite des femuralen Gelenkflächenspacers kann zudem Ausstülpungen aufweisen, um teilweise defekte bzw. teilresezierte Menisken auszufüllen. Die Randkontur kann jedoch abgerundet sein (Fig. 16, Detail W). Zudem kann der Gelenkflächenspacer randseitig aus feinfibrillärer Struktur (Fig. 16) bestehen. Optional kann die innere Randkontur eine Randfläche aufweisen (Fig. 17), so dass sich die ineinander übergehende tibiale und femurale Randkontur nur über etwa 270° erstreckt. Die femurale Form dieses Gelenkflächenspacers ergibt sich in ähnlicher Weise wie die femurale Seite des Meniskusspacers und ist konkav. Die tibiale Form ist konvex.

In einer alternativen Ausgestaltung des Gelenkflächenspacers als "tibialer" Gelenkflächenspacer liegt dieser im Wesentlichen in einer relativ flachen Form bzw. als Scheibe mit nahezu gleichmäßiger Dicke vor, der unter den Meniskus gelegt wird. Fig. 18 zeigt einen lateralen tibialen Gelenkflächenspacer 180. In medial-lateraler Richtung kann der Querschnitt jedoch auch spitz-keilförmig sein. Die tibiale Fläche dieses Gelenkflächenspacers 180 ergibt sich in ähnlicher Weise wie die tibiale Fläche des Meniskusspacers. Auch diese Ausführung des Gelenkflächenspacers kann randseitig eine feinfibrilläre Struktur aufweisen. Die randseitige feinfibrilläre Struktur ermöglichst es, dass der femurale oder tibiale Gelenkflächenspacer entweder an der oberen oder unteren Erhebung mit dem Meniskus oder der Kapselstruktur fixiert bzw. vernäht werden kann. Beide Formen des Gelenkflächenspacers können auch zur Begradigung von Abwinklungen des Beines (Valgus, Varus) verwendet werden.

Der Meniskus- bzw. Gelenkflächenspacer und insbesondere deren Materialausgestaltungen und Sandwichstrukturen sind mit entsprechender Formgebung auch als scheibenförmige Gleitfläche für uni- oder bikondyläre Endoprothesen geeignet, welche bisher üblicherweise aus Polyethylen bestehen. Vorteilhaft sind hier insbesondere die Elastizität und Dämpfungseigenschaften der Gleitflächen gegenüber den sehr harten Gleitflächen nach dem Stand der Technik. Im Falle einer bikondylären Prothese sind zwei (mediale und laterale) Scheiben gleichfalls wie bei einer Endoprothese jeweils an ihrem inneren Rand verbunden. Die Unterseite dieser Scheiben ist üblicherweise eben, sie kann auch anders, beispielsweise konvex geformt sein. Für diese Anwendung ist die Sandwichstruktur auch in der Art vorstellbar, dass eine besonders weiche Kernschicht mit härteren Deckschichten kombiniert wird, wobei die Deckschichten hierbei besonders gleitfähig sind.

Der Hüftspacer 190 (Fig. 19 ff.) ist ein elastischer Überzug für den Hüftkopf 191, der vorzugsweise ohne jegliche Knochen- und mit nur minimaler Geweberesezierung minimal-invasiv eingesetzt wird. Fig. 19 zeigt schematisch das Aufbringen des Hüftspacers 190 um den Hüftkopf 191. Der Hüftspacer 190 besitzt ungefähr am proximalen Pol eine runde oder ellipsenförmige Öffnung 192 für die Durchführung des Hüftkopfbandes 193. Durch die Öffnung am distalen Pol führt die jenseits des Äquators distale Seite des Hüftkopfes 191 bzw. der Hals des Femurs. Nach unten (caudal) ist der Hüftspacer 190 ähnlich eines "Hosenbundschlitzes" aufgetrennt (Pfeil 194), um den Hüftspacer 190 um den Hüftkopf 191 zu stülpen, wenn das Hüftkopfband 193 nicht abgetrennt wurde. Der Hüftspacer 190 ist im Bereich des Hosenbundschlitzes entlang eines Meridanbogenstreifens in seiner Dicke getrennt, vorzugsweise halbiert. Die äußere Hälfte besitzt nach innen eine Vielzahl von Noppen 195, die in Öffnungen 196 der inneren Hälfte greifen und damit ähnlich einer Knopflochleiste den Hüftspacer 190 um den Hüftkopf 191 schließen und fixieren. Wie in Fig. 19 und 20 dargestellt ist, sind die Noppen 195 in zwei Reihen entlang des Medianbogens angeordnet. Da der Hüftspacer 190 vorzugsweise sehr elastisch ausgeführt ist, kann er bezogen auf den Hüftkopf 191 ein leichtes Untermaß aufweisen, um den Hüftspacer 190 damit form- und kraftschlüssig mit dem Hüftkopf 191 zu fixieren.

In einer alternativen konkreten Ausgestaltung kann der Hüftspacer 190 proximal geschlossen sein und/oder auch ohne Schlitz ausgeführt sein, wenn das Hüftkopfband abgetrennt wurde.

Die proximale Hälfte des Hüftspacers 190 ist näherungsweise außen vorzugsweise kugelförmig und innen leicht deformiert kugelförmig bis ellipsoidförmig. Über den Äquator nach distal hinaus verjüngt sich der Hüftspacer außen ellipsoidförmig, nach innen je nach Schnittebene wieder leicht deformiert kugelförmig bis ellipsoidförmig. Die Hüftspacergeometrie ist außen vorzugsweise aus einer kugel- und einer kreisförmigen-ellipsoiden Halbschale zusammengesetzt. Beispielhafte Durchmesser liegen hier bei 58mm für den kreis- bzw. kugelförmigen Teil und 32mm für die kurze Achse des Ellipsoids. Andere absolute Größen und auch Verhältnisse der Durchmesser sind natürlich möglich. Insgesamt ergibt sich eine ungleichmäßig verteilte Dicke des Überzugs, wobei auch eine gleichmäßige Dicke möglich ist. Im Falle des proximal und caudal geschlossenen Hüftspacers ist dieser in Nähe des proximalen Pols innen abgeflacht, entsprechend der natürlichen Abflachung des Hüftkopfes, aus der das Hüftkopfband entspringt.

Der Hüftspacer 190 und damit seine Geometrie und Dickenverteilung kann optional auch individuell an den Patienten angepasst werden. Dadurch könnten Inkongruenzen von Hüftkopf und -pfanne, insbesondere von abgelaufenen Beckenpfannen, besser ausgeglichen werden. Hierbei kann zudem die distal des Äquators befindliche innere Fläche des Hüftspacers von einer kreisrunden Form abweichen, um sich dem Hüftkopfhals besser anzuschmiegen (Fig. 20 c). Die distale Hälfte des Hüftkopfspacers kann ferner bis auf den kleinsten Umfang des Femurhalses führen oder vorher abschließen, wie es in den Fig. 19 und 20 dargestellt ist.

In Fig. 20 ist zudem die Sandwichstruktur des Hüftspacers dargestellt. Um möglichst viel Spielraum für den Kongruenzausgleich zwischen Hüftkopf und -pfanne zu erzielen, welches vorzugsweise durch das weiche Material gegeben ist, kann die harte Kernschicht nur auf eine Teilfläche des Hüftkopfes beschränkt sein, die besonders stark belastet ist. Vorzugsweise ist daher die Kernschicht in Form eines Kugelzweiecks 200 ausgelegt. Der Öffnungswinkel des Kugelzweiecks der besonders tragfähigen Kernschicht ist kleiner 125°, vorzugsweise kleiner 105°. Hierbei geht die Kernschicht distal über den Äquator hinaus, proximal beginnt sie im Bereich der proximalen Öffnung, so dass die Verschlussleiste des Hüftkopfspacers vorzugsweise vollständig aus dem weicheren Material besteht. Dadurch können insbesondere die Druckspitzen bei unvollständig überdachten Hüftgelenken abgefedert werden, wo der maximale Druck sehr nah zum Pfannenrand verschoben sein kann. Die höchstbelasteten Teile des Gelenkes werden gestützt, gleichzeitig wird durch die sehr weiche caudale Hüftspacerhälfte der Kongruenzausgleich gefördert.

Zuvor wurden bereits verschiedene Ausführungsformen zum Schließen und Fixieren des aufgetrennten Hüftspacers beschrieben. In Fig. 21 sind bevorzugte Ausführungsformen dargestellt. In Fig. 21 a (obere Hälfte) ist die schräge Hakenblattung 211 im Querschnitt des Hüftspacers 190 dargestellt, wodurch sich auch ein Überlappungsbereich der Trennung ausbildet. Statt aus 2 Haken kann diese auch nur aus einem Haken bestehen. Die Hakenblatt-Verbindung 211 kann auch als vorgefertigtes Bauteil im Abformprozess (beispielsweise beim Spritzgießen) des Hüftspacers 190 in das Formnest eingelegt werden oder nachträglich an den Hüftspacer 190 angeklebt werden. In Fig. 21 a (untere Hälfte) ist eine Trennung des Hüftspacers 190 in Form eines schrägen Schnittes 212 dargestellt, die Trennflächen bzw. der Überlappungsbereich können hierbei geklebt oder geschweißt, mittels eines Klettverschlusses, mittels Heftklammern oder mehrerer durchführbarer Noppen verbunden werden. In Fig. 21b ist die Verzahnung aus Zinken in Puzzleform (Pfeil 213) dargestellt. Durch die Form der Hinterschneidungen kann man die Enden, also die Zinken, zusammenschieben und muss sie nicht - wie bei einem gewöhnlichen Puzzle - von oben zusammenstecken.

## Patentansprüche

1. Elastischer Gelenkspacer,
**dadurch gekennzeichnet, dass**
der Gelenkspacer zumindest teilweise aus einem Material, insbesondere einem Polymer, Elastomer oder thermoplastischen Elastomer, mit
- einer Shore-A Härte zwischen 20 und 77 und Zugspannungswerten bei Dehnungen zwischen 20% und 60%, vorzugsweise bei 50%, größer 3,8 N/mm², vorzugsweise größer 4,6 N/mm² und besonders bevorzugt größer 6 N/mm², und/oder Druckspannungswerten bei Stauchungen zwischen 20% und 60%, vorzugsweise bei 50%, größer 7,8 N/mm², vorzugsweise größer 9 N/mm² und besonders bevorzugt größer 10,5 N/mm², und/oder
- einer Shore-A Härte bis 85 und Zugspannungswerten bei Dehnungen zwischen 20% und 60%, vorzugsweise bei 50%, größer 6 N/mm², vorzugsweise größer 7 N/mm² und besonders bevorzugt größer 8 N/mm², und/oder Druckspannungswerten bei Stauchungen zwischen 20% und 60%, vorzugsweise bei 50% größer 10,5 N/mm², vorzugsweise größer 12 N/mm² und besonders bevorzugt größer 14 N/mm² besteht.

2. Gelenkspacer nach Anspruch 1, **dadurch gekennzeichnet, dass** das Material ein Elastomer oder thermoplastisches Elastomer ist und vorzugsweise aus der Klasse der Polyurethane besteht, wobei das Elastomer oder thermoplastische Elastomer als Weichsegmente vorzugsweise ausschließlich hydroxyl- und/oder aminterminiertes Polyisobutylen und/oder hydroxyl- und/oder aminterminiertes und bevorzugt hydroxylterminiertes hydrogeniertes Polybutadien enthält.

3. Gelenkspacer nach einem der Ansprüche 1 oder 2, **dadurch gekennzeichnet, dass** das Elastomer oder thermoplastische Elastomer als Weichsegment
a) zumindest teilweise aminterminiertes Polyisobutylen und als Kettenverlängerer Ethylendiamin und/oder 1,4-Diaminobutan und/oder besonders bevorzugt 1,6-Diaminohexan und/oder 1,8-Diaminooctan oder
b) zumindest teilweise hydroxylterminiertes Polyisobutylen und als Kettenverlängerer zumindest teilweise Hexandiol und/oder zumindest teilweise Butandiol in Kombination mit der Verwendung des Katalysators 1,3-Diacetoxy-1,1,3,3-Tetrabutyldistannoxan oder
c) zumindest teilweise hydroxylterminiertes und bevorzugt hydroxylterminiertes hydrogeniertes Polybutadien und als Kettenverlängerer zumindest teilweise 2,2,4-Trimethyl-1,3-Pentandiol und/oder 2-Butyl-2-Ethyl-1,3-Propandiol und/oder bevorzugt N,N-Diisopropanol Anilin und/oder 2-Ethyl-1,3-Hexandiol
enthält.

4. Gelenkspacer nach einem der Ansprüche 1 bis 3, **dadurch gekennzeichnet, dass** das Elastomer oder thermoplastische Elastomer als Weichsegmente zumindest teilweise Polycarbonatdiol und bevorzugt Polycarbonatdiol-Copolymertypen enthält, wobei diese auf Basis der Einheiten Hexan (C₆), Pentan (C₅), Butan (C₄) oder Propan (C₃) besonders bevorzugt als Kombinationen aus C₆/C₅ oder C₆/C₄ oder C₄/C₃ entsprechend der chemischen Summenformel 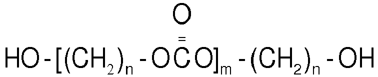 aufgebaut sind.

5. Gelenkspacer nach einem der Ansprüche 1 bis 4, **dadurch gekennzeichnet,**
a) **dass** das Elastomer oder thermoplastische Elastomer als Weichsegmente Polyisobutylen und/oder Polybutadien und ein Polycarbonatdiol und bevorzugt ein Polycarbonatdiol-Copolymertyp mit einem Anteil des Polycarbonatdiol am Gesamtweichsegmentanteil von bis zu 80%, vorzugsweise 5 bis 40%, enthält, und/oder
b) **dass** das Weichsegment oder das Gemisch der Weichsegmente des Elastomers oder thermoplastischen Elastomers die Weichsegmente Polydimethylsiloxan oder Polytetramethylenoxid oder Polydimethylsiloxan-Polycaprolacton-Blockcopolymere oder ein Gemisch einzelner oder mehrerer dieser Weichsegmente enthalten, und zwar mit einem Anteil eines oder mehrerer dieser Weichsegmente am Gesamtweichsegmentanteil von bis zu 50%, vorzugsweise von 3 bis 35%.

6. Gelenkspacer nach einem der Ansprüche 1 bis 5, **dadurch gekennzeichnet, dass** das Polyurethan zumindest teilweise aus Naphthalin-1,5-Diisocyanat und/oder Para-Phenylendiisocyanat und/oder trans-1,4-Cyclohexandiisocyanat und/oder Hexamethylendiisocyanat besteht.

7. Gelenkspacer nach einem der Ansprüche 1 bis 6, **dadurch gekennzeichnet, dass** das Polyurethan vernetzt und das Vernetzungsreagenz vorzugsweise Wasser oder Wasser in Kombination mit einem oder mehreren weiteren Kettenverlängerern und/oder Vernetzungsreagenzien ist.

8. Gelenkspacer nach einem der Ansprüche 1 bis 7, **dadurch gekennzeichnet, dass**
a) der Gelenkspacer Nanoadditive aufweist, die bevorzugt scheibenförmig oder flach ausgestaltet sind und vorzugsweise eine Breite von 10 nm bis 50 nm, vorzugsweise von 25 nm bis 30 nm, und eine Dicke von 0,5 nm bis 1,5 nm, vorzugsweise von 1 nm, besitzen, und/oder
b) der Gelenkspacer zumindest teilweise eine poröse Struktur mit Porengrößen zwischen 0,1 nm bis 2 µm, vorzugsweise zwischen 1 nm bis 500 nm und besonders bevorzugt zwischen 5 nm bis 200 nm besitzt, und/oder
c) der Gelenkspacer eine Nano-Vlies-Struktur aufweist, die aus Fasern mit einem Durchmesser von 0,1 µm bis 0,4 µm besteht.

9. Gelenkspacer nach einem der Ansprüche 1 bis 8, **dadurch gekennzeichnet, dass** der Gelenkspacer als Meniskusspacer ausgestaltet ist und dessen äußerer Rand an einer Stelle in einem Bereich von 90°, vorzugsweise in einem Bereich von 45°, gleich dick oder bevorzugt dünner als der zentrale Bereich des Meniskusspacers ist, wobei die Winkelbereiche von dem Mittelpunkt der kreisförmigen Randkontur gemessen sind.

10. Gelenkspacer nach einem der Ansprüche 1 bis 9, **dadurch gekennzeichnet, dass** der Gelenkspacer entlang seines Randes zumindest teilweise von einer porösen Vliesschicht oder einem porösen Band oder Schlauch aus feinfibrillärer Struktur oder einem zusätzlichen Fixierungsband umfasst ist, das vorzugsweise im Schlauch oder unter dem Band angeordnet ist, wobei der Rand oder bevorzugt die poröse Randschicht des Gelenkspacers vorzugsweise mit für bildgebende Verfahren sichtbaren Markern in Form kleiner Pins, feiner Fäden oder Drähte, oder einer ferromagnetischen Schicht durchzogen oder verbunden ist.

11. Gelenkspacer nach einem der Ansprüche 1 bis 10, **dadurch gekennzeichnet, dass** der Gelenkspacer als Kniespacer ausgestaltet ist und das Band, der Schlauch oder das Fixierungsband mit den Resten von Meniskushörnern oder in Bohrungen auf dem Tibiakopf verbindbar ist, oder in einer Aussparung oder einer Profilschiene oder C-förmig um den Gelenkspacer verläuft oder mit Kupplungselementen versehen ist, so dass der Gelenkspacer lösbar vom Fixierungsband ist.

12. Gelenkspacer nach einem der Ansprüche 1 bis 11, **gekennzeichnet durch** eine oder mehrere, vorzugsweise zwei, Auskragungen, die am inneren Rand angeordnet sind und mit den Einmuldungen auf dem Tibiaplateau vorzugsweise form- oder kraftschlüssig verbindbar sind, oder durch stiftförmige Auskragungen, die in vorgefertigte Bohrungen an den Einmuldungen auf dem Tibiaplateau einführbar sind.

13. Gelenkspacer nach einem der Ansprüche 1 bis 12, **dadurch gekennzeichnet, dass** der Gelenkspacer als Hüftspacer ausgestaltet ist und in Form eines schalenförmigen elastischen Überzugs über den Hüftkopf stülpbar ist, wobei vorzugsweise eine Öffnung für die Durchführung des Hüftkopfbandes vorgesehen ist.

14. Gelenkspacer nach einem der Ansprüche 1 bis 13, **dadurch gekennzeichnet, dass**
a) der Hüftspacer von der Öffnung bis zum Rand des schalenförmigen Überzugs aufgetrennt ist und entlang der Trennungslinie ein Überlappungsbereich vorgesehen ist, wobei der äußere Teil des Überlappungsbereiches vorzugsweise eine Vielzahl von Noppen aufweist, die in korrespondierende Ausnehmungen des unteren Teils des Überlappungsbereiches einführbar sind, oder
b) der Hüftspacer vom proximalen Pol zum freien Rand zumindest teilweise aufgetrennt ist, vorzugsweise reißverschlussartig mit Zinken in einer Puzzleform.

15. Gelenkspacer nach einem der Ansprüche 1 bis 14, **dadurch gekennzeichnet, dass**
a) die Randzone des Gelenkspacers zumindest teilweise aus einem Material mit einer Shore-A-Härte zwischen 20 und 77 besteht und das Material des zentralen (härteren) Bereichs eine Shore-A-Härte größer 77 mit Zugspannungswerten bei Dehnung zwischen 20% und 60%, vorzugsweise bei 50%, größer 3,8 N/mm², vorzugsweise größer 4,6 N/mm² und besonders bevorzugt größer 6 N/mm² aufweist, oder
b) der Gelenkspacer einen zumindest teilweise schichtartigen Aufbau mit mindestens drei Schichten aufweist, nämlich mindestens zwei Deckschichten und einer dazwischen angeordneten Kernschicht, die aus unterschiedlich harten Materialien bestehen, wobei die Differenz der Shore-A-Härten zwischen Deck- und Kernschicht größer als 5 und vorzugsweise zwischen 10 und 20 liegt und zumindest eine der Schichten aus einem Material mit Zugspannungswerten bei Dehnungen zwischen 20% und 60 %, vorzugsweise bei 50%, größer 3,8 N/mm², vorzugsweise größer 4,6 N/mm² und besonders bevorzugt größer 6 N/mm² und/oder Druckspannungswerten bei Stauchungen zwischen 20% und 60 %, vorzugsweise bei 50%, größer 7,8 N/mm², vorzugsweise größer 9 N/mm² und besonders bevorzugt größer 10,5 N/mm² besteht.

## Claims

1. Elastic joint spacer,
**characterized in that**
the joint spacer consists at least partially of a material, particularly a polymer, elastomer or thermoplastic elastomer, with
- a Shore-A hardness of 20-77 and tensile stress values at elongations between 20% and 60%, preferably 50%, greater than 3.8 N/mm², preferably greater than 4.6 N/mm² and more preferably greater than 6 N/mm², and/or compressive stress values at compressions between 20% and 60%, preferably 50%, greater than 7.8 N/mm², preferably greater than 9 N/mm² and more preferably greater than 10.5 N/mm², and/or
- a Shore-A hardness of up to 85 and tensile stress values at elongations between 20% and 60%, preferably 50%, greater than 6 N/mm², preferably greater than 7 N/mm² and more preferably greater than 8 N/mm², and/or compressive stress values at compressions between 20% and 60%, preferably 50%, greater 10.5 N/mm², preferably greater than 12 N/mm² and more preferably greater than 14 N/mm².

2. Joint spacer according to claim 1, **characterized in that** the material is an elastomer or thermoplastic elastomer and preferably consists of a class of polyurethanes, whereby the elastomer or thermoplastic elastomer preferably exclusively contains hydroxyl- and/or amine-terminated polyisobutylene and/or hydroxyl- and/or amine-terminated and preferably hydroxyl-terminated hydrogenated polybutadiene as soft segments.

3. Joint spacer according to one of claims 1 to 2, **characterized in that** the elastomer or thermoplastic elastomer contains
a) at least partially amine-terminated polyisobutylene as soft segment, and Ethylenediamine and/or 1,4-Diaminobutane and/or more preferably 1, 6-Diaminohexane and/or 1,8-Diaminooctane as chain extender, or
b) at least partially a hydroxyl-terminated polyisobutylene as soft segment, and at least partially hexanediol and/or at least partially butanediol as chain extender in combination with the use of the catalyst 1,3- Diacetoxy-1,1,3,3-tetrabutyldistannoxane, or
c) at least partially hydroxyl-terminated, and preferably hydroxyl-terminated hydrogenated polybutadiene as soft segment, and at least partially 2,2,4-Trimethyl-1,3-pentanediol and/or 2-Butyl-2-ethyl-1,3-propanediol and/or, preferably N,N-Diisopropanol aniline and/or 2-Ethyl-1,3-hexanediol as chain extender.

4. Joint spacer according to one of claims 1 to 3, **characterized in that** the elastomer or thermoplastic elastomer contains at least partially polycarbonate diol and preferably polycarbonate diol copolymer types as soft segments, wherein on the basis of the units hexane (C₆), pentane (C₅), butane (C₄) or propane (C₃) these are build particularly preferred as combinations of C₆/C₅ or C₆/C₄ or C₄/C₃ according to the chemical molecular formula

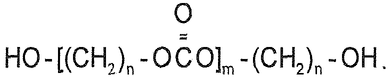

5. Joint spacer according to one of claims 1 to 4, **characterized in that**
a) the elastomer or thermoplastic elastomer contains polyisobutylene and/or polybutadiene and a polycarbonate diol as soft segments and preferably a polycarbonate diol copolymer type with a proportion of the polycarbonate diol in the total soft segment proportion of up to 80%, preferably 5 to 40%, and/or
b) the soft segment or the mixture of the soft segments of the elastomer or thermoplastic elastomer contains the soft segments polydimethylsiloxane or polytetramethylene oxide or polydimethylsiloxane-polycaprolactone block copolymers, or a mixture of one or more of these soft segments, in a proportion of one or more of these soft segments in the total soft segment proportion of up to 50%, preferably from 3 to 35%.

6. Joint spacer according to one of claims 1 to 5, **characterized in that** the polyurethane consists at least partially of Naphthalene-1,5-diisocyanate and/or para-Phenylene diisocyanate and/or trans-1,4-Cyclohexane diisocyanate and/or Hexamethylene diisocyanate.

7. Joint spacer according to any one of claims 1 to 6, **characterized in that** the polyurethane is cross-linked and the cross-linking reagent is preferably water or water in combination with one or more chain extenders and/or cross-linking reagents.

8. Joint spacer according to any one of claims 1 to 7, **characterized in that** the joint spacer has
a) nanoadditives which are designed preferably disk-shaped or flat, and preferably has a width of 10 nm to 50 nm, preferably from 25 nm to 30 nm, and a thickness of 0.5 nm to 1.5 nm, preferably 1 nm, and/or
b) at least partially a porous structure with pore sizes between 0.1 nm to 2 µm, preferably between 1 nm to 500 nm and more preferably between 5 nm to 200 nm, and/or
c) a nano-fabric structure that consists of fibers having a diameter of 0.1 to 0.4 µm.

9. Joint spacer according to any one of claims 1 to 8, **characterized in that** the joint spacer is configured as meniscus spacer, whose outer edge at a place in a range of 90°, preferably in a range of 45°, is preferably of the same thickness or is thinner than the central region of the meniscus spacer, where the said angular regions are measured from the centre of the circular edge contour.

10. Joint spacer according to any one of claims 1 to 9, **characterized in that** along its edge the joint spacer consists at least partially of a porous fleece layer or a porous tape or hose having a fine fibrillary structure or an additional fixing tape, preferably arranged in the hose or under the tape, whereby the edge or, preferably, the porous edge layer of the joint spacer is preferably traversed or connected with visible markers for imaging in the form of small pins, fine threads or wires, or a ferromagnetic layer.

11. Joint spacer according to any one of claims 1 to 10, **characterized in that** the joint spacer is configured as knee spacer and the tape, the hose or the fixing tape can be connected to the remaining meniscal horns or in bores in the tibial plateau, or runs in a recess or a profile rail or in C-shape around the joint spacer or is provided with coupling elements so that the joint spacer can be detached from the fixing tape.

12. Joint spacer according to any one of claims 1 to 11, **characterized by** one or more, preferably two, protrusions which are arranged at the inner edge and which can be connected to the trough in the tibial plateau preferably through form-fit or force-fit, or by pin-shaped projections which can be inserted into prefabricated holes in the trough configurations on the tibial plateau.

13. Joint spacer according to any one of claims 1 to 12, **characterized in that** the joint spacer is configured as hip spacer and can be slipped over the head of the femur in the form of a shell-shaped elastic coat, whereby preferably provision is made of an opening for the implementation of the femoral head ligament.

14. Joint spacer according to any one of claims 1 to 13, **characterized in that** the hip spacer is separated
a) from the opening up to the edge of the shell-shaped coat, wherein an overlapping region is provided along the separation line, whereby the outer part of the overlapping region preferably consists of a plurality of knobs that are inserted in corresponding recesses in the lower part of the overlapping region, or
b) from the proximal pole to the free edge at least partially, preferably like a zipper with tines in puzzle form.

15. Joint spacer according to any one of claims 1 to 14, **characterized in that**
a) the edge zone of the joint spacer consists at least partially of a material having a Shore-A hardness between 20 and 77 and the material of the central (harder) area has a Shore-A hardness greater than 77 N/mm² with tensile stress values at elongation between 20% and 60%, preferably 50%, greater than 3.8 N/mm², preferably greater than 4.6 and more preferably greater than 6 N/mm², or
b) the joint spacer has an at least partially layered structure with at least three layers, namely at least two cover layers and an intervening core layer made of different hard materials, wherein the difference in the Shore-A hardness between the cover and the core layer is greater than 5 and preferably between 10 and 20 and at least one of the layers is of a material with tensile stress values at elongations between 20% and 60%, preferably 50%, greater than 3.8 N/mm², preferably greater than 4.6 N/mm² and more preferably greater than 6 N/mm² and/or compressive stress values at compressions between 20% and 60%, preferably 50%, greater than 7.8 N/mm², preferably greater than 9 N/mm² and more preferably greater than 10.5 N/mm².

## Revendications

1. Spacer articulaire élastique,
**caractérisé en ce que**
le spacer articulaire se compose au minimum en partie d'un matériau, notamment d'un polymère, d'un élastomère ou d'un élastomère thermoplastique, d'une
- dureté Shore A entre 20 et 77 et doté de valeurs de contrainte de traction en dilatation se situant entre 20 % et 60 %, de préférence à 50 %, supérieures à 3,8 N/mm², de préférence supérieures à 4,6 N/mm² et particulièrement de préférence supérieures à 6 N/mm², et/ou doté de valeurs de contrainte de compression en tassement se situant entre 20 % et 60 %, de préférence à 50 % supérieures à 7,8 N/mm², de préférence supérieures à 9 N/m² et particulièrement de préférence supérieures à 10,5 N/mm², et/ou
- d'une dureté Shore A jusqu'à 85 et doté de valeurs de contrainte de traction en dilatation se situant entre 20 % et 60 %, de préférence à 50 %, supérieures à 6 N/mm², de préférence supérieures à 7 N/mm² et particulièrement de préférence supérieures à 8 N/mm², et/ou doté de valeurs de contrainte de compression en tassement se situant entre 20 % et 60 %, de préférence à 50 %, supérieures à 10,5 N/mm², de préférence supérieures à 12 N/mm² et particulièrement de préférence supérieures à 14 N/mm².

2. Spacer articulaire selon revendication 1, **caractérisé en ce que** le matériau est un élastomère ou un élastomère thermoplastique et de préférence de la classe des polyuréthanes, l'élastomère ou l'élastomère thermoplastique en tant que segment mou contenant de préférence exclusivement du polyisobutylène à terminaison hydroxyle et/ou à terminaison amine et/ou du polybutadiène à terminaison hydroxyle et/ou amine et de préférence un polybutadiène hydrogéné à terminaison hydroxyle.

3. Spacer articulaire selon l'une des revendications 1 ou 2, **caractérisé en ce que** l'élastomère ou l'élastomère thermoplastique en tant que segment mou
a) contient au moins partiellement du polyisobutylène à terminaison amine et en tant qu'agent d'allongement de chaine de l'éthylènediamine et/ou du 1,4-diaminobutane et/ou notamment de préférence de l'hexaméthylènediamine et/ou du 1,8-diaminooctane ou
b) au moins en partie du polyisobutylène à terminaison hydroxyle et en tant qu'agent d'allongement de chaine au minimum en partie de l'hexanediol et/ou au moins en partie du butanediol en combinaison avec l'utilisation du catalyseur 1,3-diacetoxy-1,1,3,3-tetrabutyldistannoxane ou
c) au minimum en partie du polybutadiène à terminaison hydroxyle et de préférence hydrogéné à terminaison hydroxyle et en tant qu'agent d'allongement de chaine au moins en partie de 2,2,4-trimethyle-pentane-1,3-diol et/ou 2-butyle-2-éthyle-propane-1,3-diol et/ou de préférence d'aniline N,N-Diisopropanol et/ou 2-éthyle-hexane-1,3-diol.

4. Spacer articulaire selon l'une des revendications 1 à 3, **caractérisé en ce que** l'élastomère ou l'élastomère thermoplastique en tant que segment mou contient au minimum en partie du polycarbonate-diol et de préférence des types de copolymères de polycarbonate-diol, ceux-ci étant structurés sur la base des unités hexane (C₆), pentane (C₅), butane (C₄) ou propane (C₃) notamment de préférence en tant que combinaisons de C₆/C₅ ou C₆/C₄ ou C₄/C₃ conformément à la formule moléculaire chimique

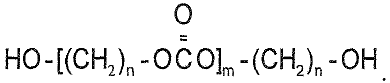

5. Spacer articulaire selon l'une des revendications 1 à 4, **caractérisé en ce que**
a) l'élastomère ou l'élastomère thermoplastique en tant que segment mou contient du polyisobutylène et/ou du polybutadiène et un polycarbonate-diol et de préférence un type de copolymère de polycarbonate-diol avec une teneur de polycarbonate-diol sur la part du segment mou total allant jusqu'à 80 %, de préférence entre 5 et 40 %, et/ou
b) le segment mou ou le mélange de segments mous de l'élastomère ou de l'élastomère thermoplastique contiennent les segments mous polydiméthylsiloxane ou oxyde de polytétraméthylène ou des copolymères en bloc de polydiméthylsiloxane-polycaprolactone ou un mélange de certains ou de plusieurs de ces segments mous, et notamment avec une teneur d'un ou plusieurs de ces segments mous sur la part du segment mou total allant jusqu'à 50 %, de préférence entre 3 et 35 %.

6. Spacer articulaire selon l'une des revendications 1 à 5, **caractérisé en ce que** le polyuréthane est composé au moins en partie de naphthalène-1,5-diisocyanate et/ou de paraphénylène diisocyanate et/ou de trans-1,4-cyclohexane diisocyanate et/ou de hexamethylene diisocyanate.

7. Spacer articulaire selon l'une des revendications 1 à 6, **caractérisé en ce que** le polyuréthane est réticulé et que le réactif de réticulation est de préférence de l'eau ou de l'eau en association avec un ou plusieurs agents d'allongement de chaine et/ou réactifs de réticulation.

8. Spacer articulaire selon l'une des revendications 1 à 7, **caractérisé en ce que**
a) le spacer articulaire présente des nanoadditifs qui de préférence sont organisés en forme de disque ou plats et qui possèdent une largeur entre 10 nm et 50 nm, de préférence entre 25 nm et 30 nm et une épaisseur entre 0,5 nm et 1,5 nm, de préférence de 1 nm, et/ou
b) le spacer articulaire possède au moins en partie une structure poreuse avec des tailles de pores entre 0,1 nm et 2 µm, de préférence entre 1 nm et 500 nm et notamment de préférence entre 5 nm et 200 nm, et/ou
c) le spacer articulaire présente une structure nano non tissée composée de fibres d'un diamètre entre 0,1 µm et 0,4 µm.

9. Spacer articulaire selon l'une des revendications 1 à 8, **caractérisé en ce que** le spacer articulaire est conçu comme spacer de ménisque et dont le bord externe sur un point dans une plage de 90°, de préférence dans une plage de 45°, est de même épaisseur ou de préférence plus fin que la plage centrale du spacer de ménisque, les plages angulaires étant mesurées depuis le point central du contour marginal circulaire.

10. Spacer articulaire selon l'une des revendications 1 à 9, **caractérisé en ce que** le spacer articulaire est au moins en partie enveloppé le long de son bord d'une couche non tissée poreuse ou d'une bande poreuse ou d'un flexible en structure de fines fibres ou d'une bande de fixation supplémentaire, placée de préférence dans le flexible ou sous la bande, le bord ou de préférence la couche marginale poreuse du spacer articulaire étant de préférence traversée ou reliée avec des marqueurs visibles pour un procédé d'imagerie sous la forme de petites broches, de fils ou fils métalliques fins, ou d'une couche ferromagnétique.

11. Spacer articulaire selon l'une des revendications 1 à 10, **caractérisé en ce que** le spacer articulaire est conçu comme spacer de genou et que la bande, le flexible ou la bande de fixation peut être relié aux restes des cornes du ménisque ou dans des perforations sur la tête du tibia, ou passe dans une cavité ou un rail profilé ou en forme de C autour du spacer de genou ou est doté d'éléments d'accouplement, de sorte que le spacer articulaire peut être détaché de la bande de fixation.

12. Spacer articulaire selon l'une des revendications 1 à 11, **caractérisé par** une ou plusieurs, de préférence deux saillies placées sur le bord intérieur et pouvant être reliées avec les jonctions sur le plateau tibial de préférence par ajustement de forme ou de force, ou par des saillies en forme de tige pouvant être insérées dans les alésages préparés sur les jonctions sur le plateau tibial.

13. Spacer articulaire selon l'une des revendications 1 à 12, **caractérisé en ce que** le spacer articulaire est conçu en tant que spacer fémoral et peut être enfoncé sous la forme d'un enrobage élastique en forme de coque au-dessus de la tête fémorale, une ouverture étant de préférence prévue pour la mise en place de la bande de la tête fémorale.

14. Spacer articulaire selon l'une des revendications 1 à 13, **caractérisé en ce que**
a) le spacer fémoral est divisé depuis l'ouverture jusqu'au bord de l'enrobage en forme de coque et une zone de chevauchement est prévue le long de la ligne de séparation, la partie extérieure de la zone de chevauchement présentant de préférence une multitude de picots, pouvant être insérés dans les encastrements correspondants de la partie inférieure de la zone de chevauchement, ou
b) le spacer fémoral est au moins en partie divisé depuis le pôle proximal jusqu'au bord libre, de préférence sous la forme d'une fermeture éclair avec des dents de la forme d'un puzzle.

15. Spacer articulaire selon l'une des revendications 1 à 14, **caractérisé en ce que**
a) la zone marginale du spacer articulaire se compose au moins en partie d'un matériau d'une dureté Shore A entre 20 et 77 et le matériau de la zone centrale (la plus dure) présente une dureté Shore A supérieure à 77 avec des valeurs de contrainte de traction à l'étirement situées entre 20 % et 60 %, de préférence à 50 % supérieures à 3,8 N/mm², de préférence supérieures à 4,6 N/mm² et particulièrement de préférence supérieures à 6 N/mm², ou
b) le spacer articulaire présente au moins en partie une structure en couches composée d'au minimum trois couches, à savoir au moins deux couches supérieures et une couche centrale placée entre elles, composées de matériaux d'une dureté différente, la différence des duretés Shore A entre la couche supérieure et la couche centrale se situant au-dessus de 5 et de préférence entre 10 et 20 et au moins une des couches se composant d'un matériau avec des valeurs de contrainte de traction à l'étirement se situant entre 20 % et 60 %, de préférence à 50 % supérieures à 3,8 N/mm², de préférence supérieures à 4,6 N/mm² et notamment de préférence supérieures à 6 N/mm² et/ou des valeurs de contrainte de compression en tassement se situant entre 20 % et 60 %, de préférence à 50 %, supérieures à 7,8 N/mm², de préférence supérieures à 9 N/mm² et notamment de préférence supérieures à 10,5 N/mm².
